# EUROPEAN PATENT APPLICATION

(11) **EP 4 261 244 A1**
(43) Date of publication of application: **18.10.2023**
(21) Application number: 22305547.6
(22) Date of filing: 14.04.2022
(51) Int. Cl.: C08G 83/00, A61K 49/18, C07F 9/38, A61K 9/00, A61K 49/00, C07C 235/48, C07F 9/40, A61K 47/00

(54) **DENDRITIC MOLECULES, PROCESS FOR THEIR PREPARATION AND USES THEREOF**

(71) Applicant: Superbranche, 68420 Hattstatt (FR)
(72) Inventor: FELDER-FLESCH, Delphine, 68420 HATTSTATT (FR); AYELA, Benjamin, 67400 ILLKIRCH GRAFFENSTADEN (FR)
(74) Representative: IPAZ

(57) **Abstract**

The present invention relates to polyfunctional organic dendritic molecules derived from alkyl phosphonates, to a process for preparing the same and to the use thereof, in particular as drug carriers or contrast agents.

## Description

### TECHNICAL FIELD

The invention belongs to the field of dendritic molecules.

More particularly, the invention relates to polyfunctional organic dendritic molecules, to a process for preparing the same and to the use thereof, in particular as drug carriers or contrast agents.

### BACKGROUND

Dendrimers and their elementary unit called "dendron", are synthetically produced as monodisperse polymeric nanostructures with a tree-like, highly branched architecture. They are routinely synthesized as tunable "nanostructures" that may be designed and regulated as a function of their size, shape, surface chemistry and interior void space. They are typically 2 to 20 nm in diameter. A variety of structures are available, and each has properties such as polyvalency, self-assembling, electrostatic interactions, chemical stability, low cytotoxicity, and solubility.

Dendrimers and dendrons offer a plethora of applications deriving from the intrinsic properties of polymers but also and especially from their characteristics: on-surface easily accessible functions, porosity, flexibility of the internal branches, presence of functionalized cavities, accessibility to the core, and of course multivalency and cooperativity. They are extremely adaptable materials, with respect to their structure, flexibility, porosity or morphology, which can all be tuned at will. Their applications rely on chemistry (synthesis, analysis, catalysis...), materials sciences (films, layers and hybrids), pharmacology (drugs, medicine), nanosciences (nanoparticles), biology, and medicine (immunology). Dendrimers and dendrons are widely investigated and utilized in biomedical applications, as they have multiple surface functional groups that can be used to target or label for imaging and drug delivery applications.

Dendrimers and dendrons have found application in transdermal drug delivery systems and show potential in gene delivery and for enhancing the oral bioavailability of problematic drugs. The presence of numerous surface groups makes dendrimers suitable carriers for delivering high drug payloads. The interior space of the branched structures can be used to conjugate or encapsulate drugs. They are utilized for delivery vehicles of nonsteroidal anti-inflammatory drugs (NSAIDs), anticancer drugs, and other drugs such as simvastatin, famotidine, or quinolones. Drug-dendrimer conjugates show high solubility, reduced systemic toxicity, and selective accumulation in solid tumors.

The multivalent character of dendrimers and dendrons also positioned these well-defined and hyper connected macromolecules to the foreground in the development of new contrast agents for medical imaging or diagnosis platforms with adjustable retention times and bio distribution properties according to their generation/size, to their flexibility and/or their hydrophilicity. In addition to chemistry, the characterization and the physicochemical properties of these structures were studied in detail.

A dendritic approach as a coating strategy for the design of functional nano-objects is particularly interesting in the field of cancer diagnostics. The appeal of such strategy is due to the unique properties of the dendritic structures which can be chemically tuned to reach ideal bio distribution or highly and efficient targeting efficacies. To improve tumor targeting efficacy and to obtain better *in vivo* imaging properties, several studies explored the multivalency effect of dendrimers or of a dendritic surface functionalization of nanomaterials. Due to their conical-like architecture and focal points, dendritic structures are of particular interest as coatings of ultrasmall nanoparticles (NPs) with very high surface curvature. Certainly, such cone shapes are expected to improve steric resistance to macromolecules such as proteins while preventing better particle agglomeration by comparison with their linear counterparts.

A first objective of the present invention is to provide a new class of dendritic molecules that makes possible the delivery and targeting of many diagnostic and/or therapeutic agents.

A second objective of the present invention is to provide a new class of dendritic molecules that can be used in phase change emulsions (PCEs) and able to (i) control the size and stabilize nanodroplets, (ii) precisely control the phase change phenomenon, (iii) obtain predetermined microbubbles sizes and size distributions, and (iv) stabilize these microbubbles.

A third objective of the present invention is also to provide a new class of dendritic molecules effective and useful in controlling the properties of nanoemulsions and microbubbles, independently of the PCE.

Finally, a fourth objective of the present invention is to provide a preparation process allowing the synthesis of this new class of dendritic molecules.

These objectives are reached by the dendritic molecules of formula (I) as described in detail thereafter and by their preparation processes.

### Description of the invention

A first object of the present invention is a dendritic molecule of formula (I) below: 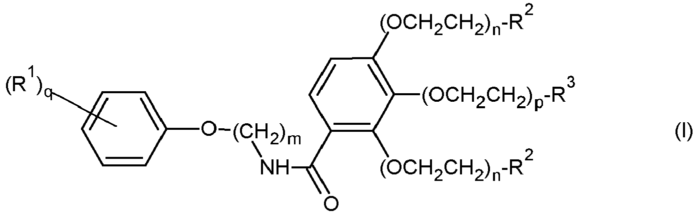 wherein :
- R¹ is a group selected among:
   ^{∗} an alkyl radical having at least 2 carbon atoms or an alkyl radical having at least 2 carbon atoms and comprising a terminal fluorinated group,
   ^{∗} a group -OR⁴ or -COOR⁴ in which R⁴ represents a linear alkyl radical having at least 4 carbon atoms or an alkyl radical having at least 2 carbon atoms and comprising a terminal fluorinated group, and
   ^{∗} a phosphonate group of the following formula (PG): 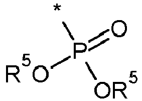 (PG) in which each of R⁵ represents a linear alkyl radical having at least 4 carbon atoms and the star represents the attachment point of said group of formula (PG) to the phenyl cycle;
- each of R² represents a linear alkyloxy radical having from 1 to 20 carbon atoms;
- R³ represents a linear alkyloxy radical having from 1 to 20 carbon atoms or a carboxyl group;
- n is an integer ranging from 1 to 16;
- p is an integer ranging from 1 to 16;
- m is an integer ranging from 1 to 4, preferably m = 1 or 2 and more preferably m = 2; and
- q is an integer ranging from 1 to 3, with the proviso that when R¹ represents a phosphonate group PG, then q = 2.

With reference to R¹, "linear alkyl radical having at least 2 carbon atoms" means a hydrocarbon group with a linear chain of at least 2 carbon atoms, preferably from 2 to 12 carbon atoms and more preferably from 2 to 8 carbon atoms. Examples of said groups are methyl, propyl, butyl, pentyl, hexyl, heptyl, octyl, nonyl, decyl, undecyl and dodecyl groups.

With reference to R¹ and R⁴, "alkyl radical having at least 2 carbon atoms and comprising a terminal fluorinated group" means a hydrocarbon group with a linear or branched chain of at least 2 carbon atoms, preferably from 2 to 12 carbon atoms, and more preferably from 2 to 8 carbon atoms, and bearing at the end of said chain, at least one fluorinated group. Examples of fluorinated groups are -CF₂-CF₃ and -CF(-CF3)2.

With reference to R⁴ and R⁵, "linear alkyl radical having at least 4 carbon atoms" means a hydrocarbon group with a linear chain of at least 4 carbon atoms, preferably from 4 to 12 carbon atoms and more preferably from 4 to 8 carbon atoms. Examples of said groups are butyl, pentyl, hexyl, heptyl, octyl, nonyl, decyl, undecyl and dodecyl groups.

With reference to R² and to R³, "linear alkyloxy radical having from 1 to 20 carbon atoms" means a hydrocarbon group with a linear chain of 1 to 20 carbon atoms, preferably from 1 to 4 carbon atoms, and more preferably having only one carbon atom, said linear chain of carbon atoms being linked to an oxygen atom. Examples of said group are methyloxy, ethyloxy, propyloxy and butyloxy groups.

According to a preferred embodiment of the present invention, n is an integer ranging from 4 to 6 inclusively and even more preferably n = 4.

According to another preferred embodiment of the present invention, p is an integer ranging from 4 to 10 inclusively and even more preferably p = 4 or p = 9.

According to a particular and preferred embodiment of the present invention, q = 2 and R¹ represents a phosphonate group of formula (PG) in which each of R⁵ represents an alkyl group having from 4 to 12 carbon atoms, more preferably R⁵ is an alkyl group selected among octyl, decanyl, and dodecanyl.

According to another particular embodiment of the present invention, q = 2 and R¹ represents an alkyl group selected among octyl, decanyl, and dodecanyl or a fluorinated group selected among -(CH₂)₆-CF₂CF₃ and -(CH₂)₂-CF(CF₃)₂.

According to another particular embodiment of the present invention, q = 2, R represents a group -OR⁴ or -COOR⁴ in which R⁴ represents an alkyl group selected among octyl, decanyl, and dodecanyl or a fluorinated group selected among -(CH₂)₆-CF₂CF₃ and -(CH₂)₂-CF(CF₃)₂.

According to a particular and preferred embodiment of the present invention, each of R² represents a methyloxy group.

According to another particular and preferred embodiment of the present invention, R³ represents a methyloxy group or a carboxyl group.

According to a most preferred embodiment of the present invention, compounds of formula (I) according to the present invention are selected among compounds of formulae (I-A) to (I-O) whose significations of R¹ to R⁵, m, n, p, and q are given in the following Table 1:

**TABLE 1**

| Cpds | **R¹** | **R²** | **R³** | **R⁴** | **R⁵** | **n** | **p** | **m** | **q** |
|---|---|---|---|---|---|---|---|---|---|
| (I-A) | PG | -OCH₃ | -OCH₃ | - | -(CH₂)₇- | 4 | 4 | 2 | 2 |
| (I-B) | PG | -OCH₃ | -OCH₃ | - | -(CH₂)₉-CH₃ | 4 | 4 | 2 | 2 |
| (I-C) | PG | -OCH₃ | -OCH₃ | - | -(CH₂)₁₁-CH₃ | 4 | 4 | 2 | 2 |
| (I-D) | PG | -OCH₃ | COOH | - | -(CH₂)₇-CH₃ | 4 | 9 | 2 | 2 |
| (I-E) | PG | -OCH₃ | COOH | - | -(CH₂)₉-CH₃ | 4 | 9 | 2 | 2 |
| (I-F) | PG | -OCH₃ | COOH | - | -(CH₂)₁₁-CH₃ | 4 | 9 | 2 | 2 |
| (I-G) | -(CH₂)₇-CH₃ | -OCH₃ | COOH | - | - | 4 | 9 | 2 | 2 |
| (I-H) | -(CH₂)₆CF₂CF₃ | -OCH₃ | COOH | - | - | 4 | 9 | 2 | 2 |
| (I-I) | -(CH₂)₂CF(CF₃)₂ | -OCH₃ | COOH | - | - | 4 | 9 | 2 | 2 |
| (I-J) | -COOR⁴ | -OCH₃ | COOH | -(CH₂)₇-CH₃ | - | 4 | 9 | 2 | 2 |
| (I-K) | -COOR⁴ | -OCH₃ | COOH | -(CH₂)₆CF₂CF₃ | - | 4 | 9 | 2 | 2 |
| (I-L) | -COOR⁴ | -OCH₃ | COOH | (CH₂)₂CF(CF₃)₂ | - | 4 | 9 | 2 | 2 |
| (I-M) | -OR⁴ | -OCH₃ | COOH | -(CH₂)₇-CH₃ | - | 4 | 9 | 2 | 2 |
| (I-N) | -OR⁴ | -OCH₃ | COOH | (CH₂)₂CF(CF₃)₂ | - | 4 | 9 | 2 | 2 |
| (I-O) | -OR⁴ | -OCH₃ | COOH | -(CH₂)₆CF₂CF₃ | - | 4 | 9 | 2 | 2 |

A second object of the present invention is a process for the preparation of the dendritic molecules of formula (I) in which R¹ is a phosphonate group of formula (PG), q = 2, n = p and R² and R³ are identical and represent an alkyloxy group as defined above in formula (I), said process comprising at least one step of reacting a compound of formula (II) below: 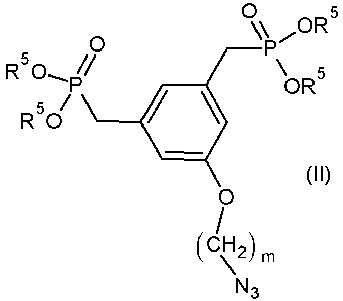 wherein R⁵ and m have the same signification as in formula (I) above, with a compound of formula (III) below: 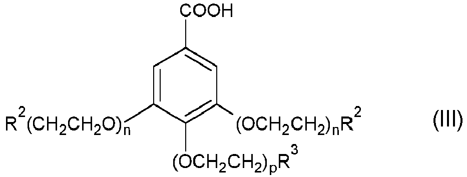 wherein n = p and have the same definition as in formula (I) above and further wherein R² and R³ are identical and represent an alkyloxy group as defined in formula (I), to lead to the corresponding compound of formula (I).

The reaction of compounds of formulae (II) and (III) can be carried out at room temperature, i.e. at a temperature ranging from 18 to 25°C, by mixing a solution of a compound of formula (II) in an appropriate solvent such as for example ethyl acetate, in the presence of a catalyst such as palladium/C, with a solution of a compound of formula (III) in an appropriate solvent such as for example dichloromethane in the presence of oxalyl chloride, dimethylformamide and N,N-diisopropylethylamine. The resulting compound of formula (I) can then be recovered and purified according to the usual practice known from one skilled in the art.

Compounds of formula (III) may be prepared according to a process comprising at least the following steps:
- reacting an alcohol of formula R⁶-(OCH₂CH₂)ₙOH (IV) in which R⁶ represents a linear alkyloxy radical having from 1 to 20 carbon atoms with tosyl chloride to obtain a compound of formula (VI) below: 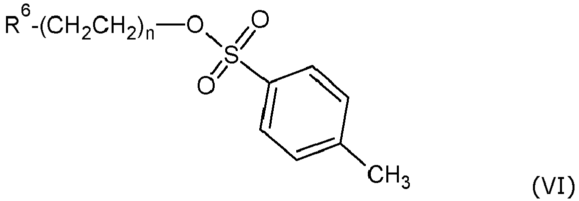 wherein R⁶ has the same meaning as in formula (IV);
- reacting said compound of formula (VI) with methyl gallate to obtain a compound of formula (VIII) below: 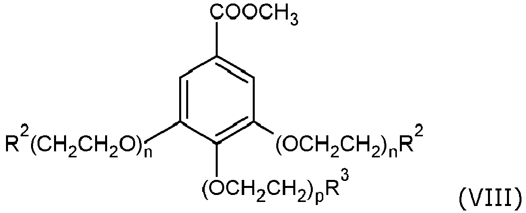 wherein n, p, R² and R³ have the same meaning as in formula (III); and
- unprotecting the carboxyl function of compound of formula (VIII) thus obtained to lead to the corresponding compound of formula (III).

The process for the preparation of compounds of formula (III) can be represented by the following Scheme 1:

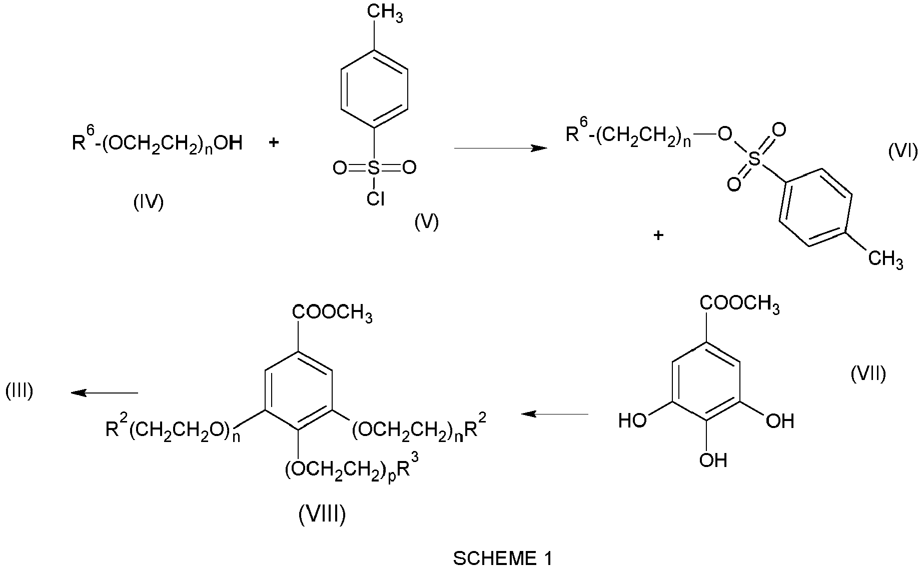

According to the process represented on Scheme 1, a solution of a compound of formula (IV) in which R⁶ has the same meaning as R² and R³ in the compounds of formula (III) above, (R² and R³ being identical) in an appropriate solvent such as for example dichloromethane in the presence of an amine such as for example triethylamine is contacted with a compound of formula (V), at room temperature under mixing until a compound of formula (VI) is obtained wherein R⁶ has the same meaning as in formula (IV) above, (R² and R³ being identical). The compound of formula (VI) is then reacted with a solution of compound of formula (VII) (methylgallate) in an appropriate solvent such as for example acetone, in the presence of potassium carbonate and potassium iodide and heated to reflux under mixing for about 8 to 16 hours to obtain a compound of formula (VIII) in which R² is identical to R³ and is an alkyl group as defined above in formula (I). The carboxyl group of compound of formula (VIII) is then unprotected by reacting said compound of formula (VIII) dissolved in an appropriate solvent such a lower alcohol, i.e. methanol or a mixture of a lower alcohol with water, in particular a mixture of methanol and water, in the presence of an alkalinizing agent such as for example sodium hydroxide, at room temperature, to lead to the corresponding compound of formula (III).

Compounds of formula (II) may be prepared according to a process comprising at least the following steps:
- reacting an alcohol of formula R⁵-OH (IX) wherein R⁵ has the same meaning as in formula (I) with trimethylphosphite (compound of formula (X)) to obtain a compound of formula (XI) below: 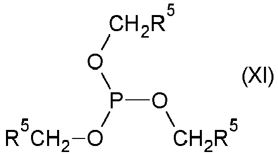 wherein R⁵ has the same meaning as in formula (I);
- reacting the compound of formula XI thus obtained with 3,5-bis(bromomethyl)phenol (compound of formula (XII)) to obtain a compound of formula (XIII) below: 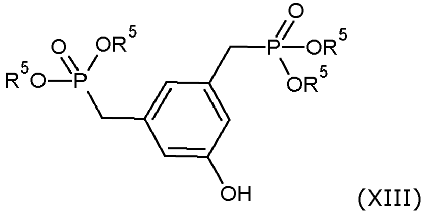 wherein R⁵ has the same meaning as in formula (I); and
- reacting the compound of formula (XIII) thus obtained with a compound of formula (XIV) below: 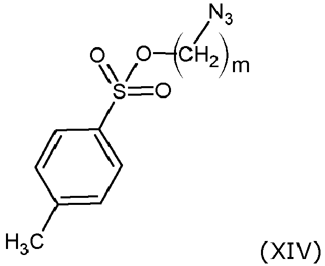 in which m has the same meaning as in formula (I) to obtain the corresponding compound of formula (II).

The process for the preparation of compounds of formula (II) can be represented by the following Scheme 2:

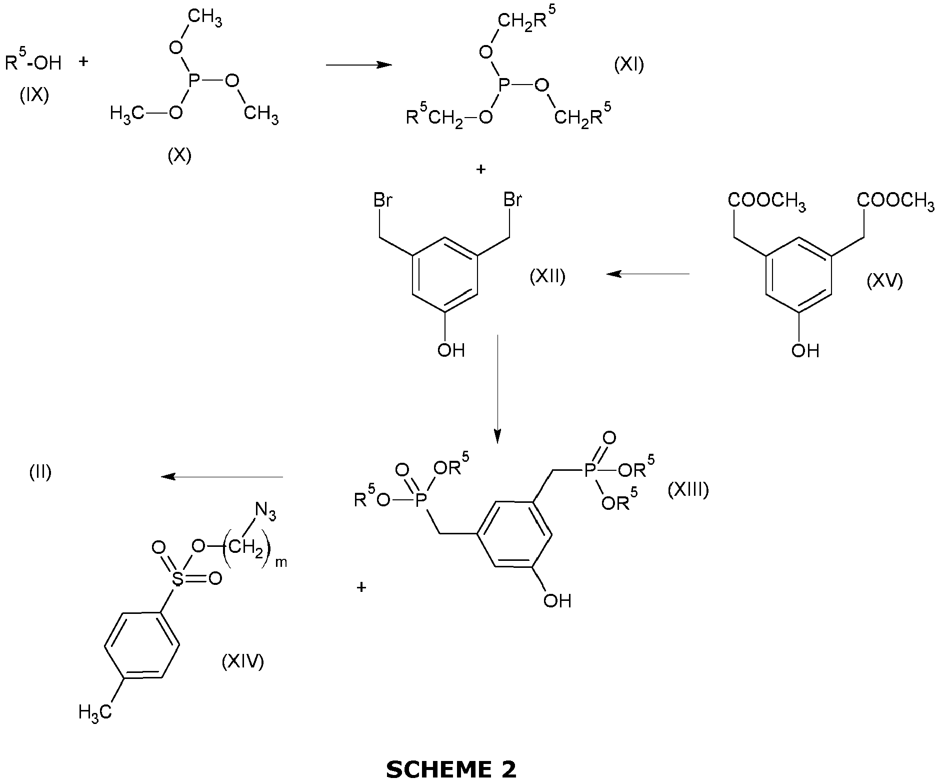

According to the process represented on Scheme 2, a compound of formula (X) (trimethylphosphite) is added to an alcohol of formula (IX) in which R⁵ has the same meaning as in formula (I) above, said alcohol of formula (IX) having previously been heated at a temperature of 30 to 75°C. The resulting mixture is then heated to a temperature of 130 to 230°C under argon atmosphere during 5 to 16 hours, to lead to compound of formula (XI) in which R⁵ has the same meaning as in formula (I) above. Compound (XI) may be separated from the remaining alcohol of formula (IX) for example by distillation. Compound of formula (XI) is then contacted with a compound of formula (XII) under stirring at a temperature of about 110 to 150°C for a period of time ranging from 8 to 16 hours to lead to compound of formula (XIII) in which R⁵ has the same meaning as in formula (I) above. A compound of formula (XIV) in which m has the same meaning as in formula (I) is added to a solution of the compound of formula (XIII) in an appropriate solvent such as for example toluene, said solution comprising an alkalinizing agent such as for example potassium hydroxide and potassium iodide and being previously heated at a temperature of 60 to 90°C. The resulting mixture is maintained at a temperature of 60 to 90°C and stirred for 8 to 16 hours to lead to a compound of formula (II) which can be recovered and purified by the usual techniques well known from one skilled in the art.

Compound of formula (XIV) can be previously prepared according to the process represented by the following Scheme 3:

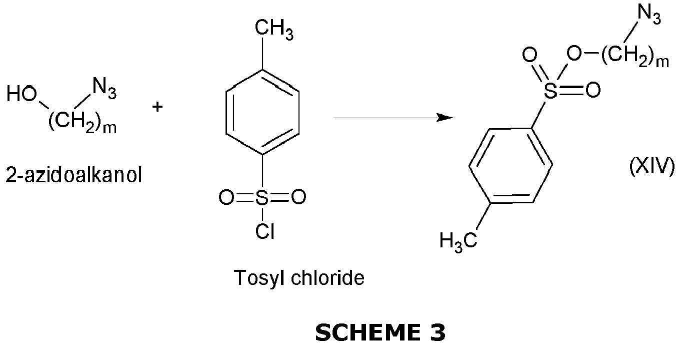

According to the process represented on Scheme 3, a solution of a 2-azidoalkanol in which m has the same meaning as in formula (I) in an appropriate solvent such as for example dichloromethane, is reacted with tosyl chloride in the presence of trimethylamine at room temperature for 8 to 16 hours to lead to a compound of formula (XIV) in which m has the same meaning as in formula (I).

A third object of the present invention is a process for the preparation of the dendritic molecules of formula (I) in which R¹ is a phosphonate group (PG), q = 2, n and p are identical or different and in which R² is an alkyloxy group as defined above in formula (I) and R³ is a carboxyl group, said process comprising at least the following steps:
- reacting methyl gallate (compound of formula (VII) as defined above) with benzyl bromide to obtain a compound of formula (XV) below: 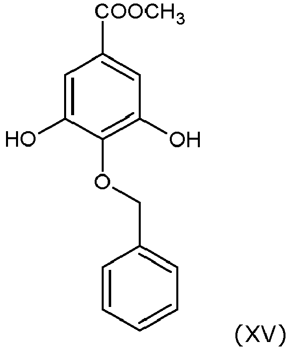
- independently reacting an alcohol of formula R²-(CH₂CH₂)ₙOH (IV') wherein R² and n have the same meaning as in formula (I) above with tosyl chloride (compound of formula (V) as defined above) to obtain a compound of formula (VI') below: 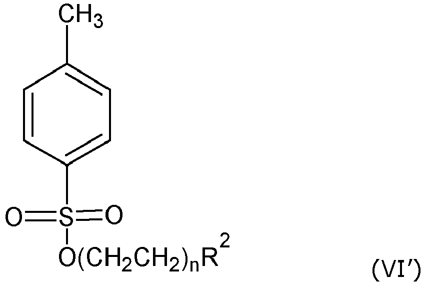 wherein R² and n have the same meaning as in formula (I) above;
- reacting the compound of formula (XV) and the compound of formula (VI') thus obtained to obtain a compound of formula (XVI) below: 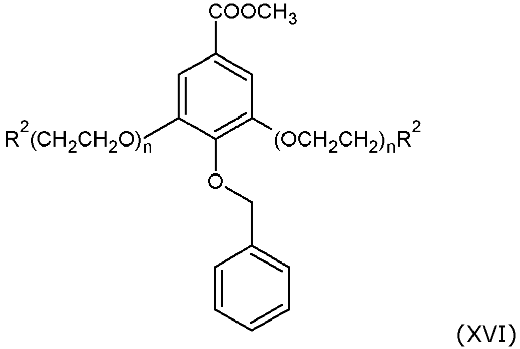 wherein R² and n have the same meaning as in formula (I) above;
- unprotecting the carboxylic function of the compound of formula (XVI) thus obtained to lead to the compound of formula (XVII) below: 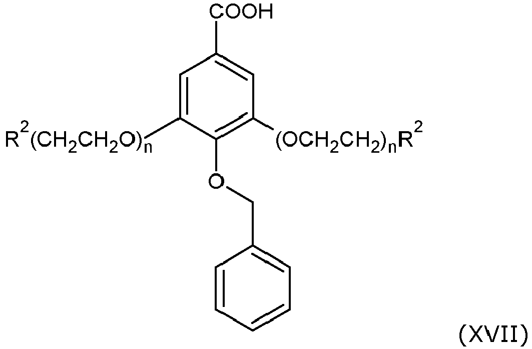 wherein R² and n have the same meaning as in formula (I) as defined above;
- reacting the compound of formula (XVII) thus obtained with a compound of formula (II) as defined above to obtain a compound of formula (XVIII) below: 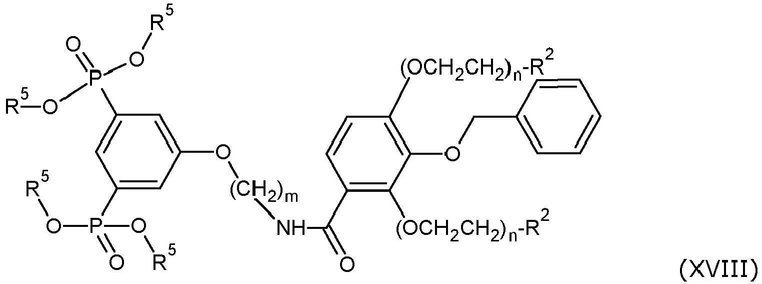 wherein R⁵, R², m and n have the same meaning as in formula (I) above;
- hydrolyzing the benzyl group of the compound of formula (XVIII) thus obtained to obtain a compound of formula (XIX) below: 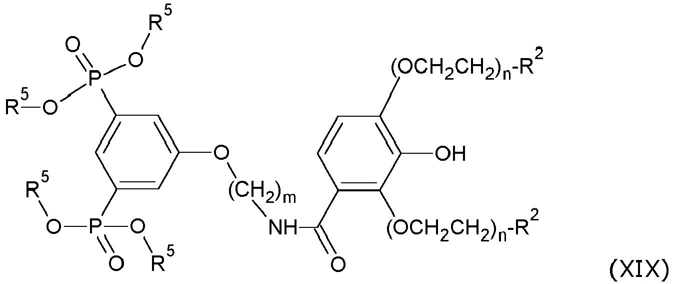 wherein R⁵, R², m and n have the same meaning as in formula (I) above;
- reacting the compound of formula (XIX) thus obtained with a compound of formula (XX) below: 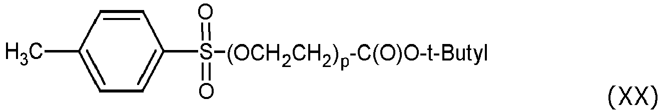 wherein p has the same meaning as in formula (I) and has a value which is identical or different to the value of n of the compound in formula (XIX) as defined above, to obtain a compound of formula (XXI) below: 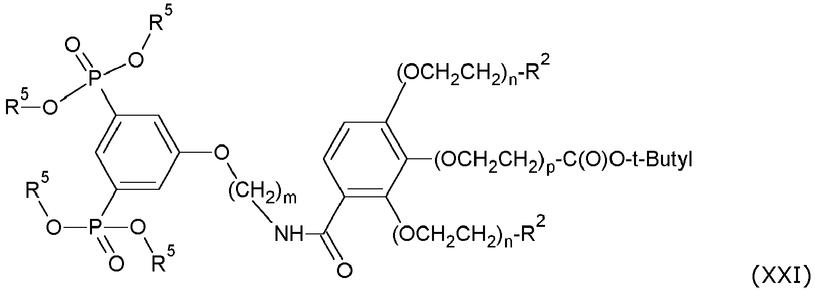 wherein R⁵, R² m, n and p have the same meaning as in formula (I) above and p has the same meaning as in formula (I) and has a value which is identical or different to the value of n in the compound of formula (XIX) as defined above;
- unprotecting the carboxylic function of compound of formula (XXI) thus obtained to lead to the corresponding dendritic molecule of formula (I).

The process for the preparation of the dendritic molecules of formula (I) in which n and p are identical or different and in which R² is an alkyloxy group as defined above in formula (I) and R³ is a carboxyl group can be represented by the following Scheme 4:

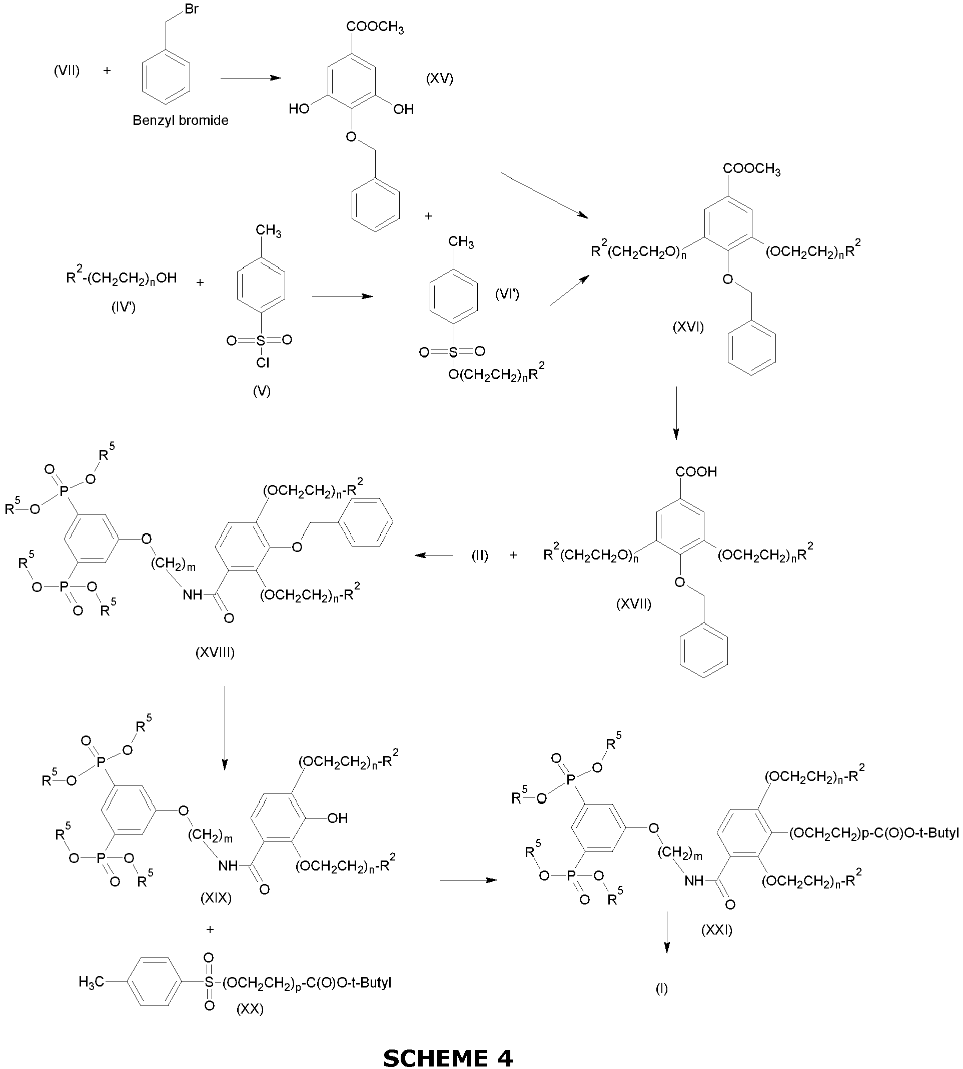

According to the process represented on Scheme 4, a compound of formula (VII) as defined above in Scheme 1 in solution in an appropriate solvent such as for example dimethylformamide is reacted with benzyl bromide in the presence of potassium hydrogen carbonate and potassium iodide at room temperature for 8 to 24 hours to lead to compound of formula (XV). Independently, a solution of a compound of formula (IV') in which R² as the same meaning as in formula (I) above, in an appropriate solvent such as for example dichloromethane in the presence of an amine such as for example trimethylamine, is contacted with a compound of formula (V), at room temperature under mixing until a compound of formula (VI') wherein R² as the same meaning as in formula (I) above is obtained. A solution of the compound of formula (XV) in an appropriate solvent such as for example acetone is then reacted with compound of formula (VI') thus obtained in the presence of an alkalinizing agent such as for example potassium carbonate, and potassium iodide. The resulting mixture is then heated at reflux for 8 to 24 hours to lead to the corresponding compound of formula (XVI) wherein R² and n have the same meaning as in the compound of formula (VI'). The carboxyl group of compound of formula (XVI) thus obtained is then unprotected by reacting said compound of formula (XVI) dissolved in an appropriate solvent such a lower alcohol, i.e. methanol or a mixture of a lower alcohol with water, in particular a mixture of methanol and water, in the presence of an alkalinizing agent such as for example sodium hydroxide, at room temperature, to lead to the corresponding compound of formula (XVII) wherein R² and n have the same meaning as in the compound of formula (XVI). The reaction of compounds of formula (II) as defined above with reference to the process represented in Scheme 2 and in which the radicals R⁵ and m have the same meaning as in formula (I) with the compound of formula (XVII) thus obtained can be carried out at room temperature, by mixing a solution of said compound of formula (II) in an appropriate solvent such as for example ethyl acetate, in the presence of a catalyst such as palladium/C, with a solution of a compound of formula (XVII) in an appropriate solvent such as for example dichloromethane in the presence of oxalyl chloride, dimethylformamide and then of N,N-diisopropylethylamine to lead to the corresponding compound of formula (XVIII) in which R⁵, R², m and n have the same meaning as in formula (I). A solution of the resulting compound of formula (XVIII) in an appropriate solvent such as for example ethyl acetate comprising a catalyst such as for example palladium/C is then purged with a hydrogen atmosphere in stirred at room temperature for 5 to 24 hours to lead to the corresponding compound of formula (XIX) in which R⁵, R², m and n have the same meaning as in formula (I). A solution of the compound of formula (XIX) thus obtained in an appropriate solvent such as for example acetone is then contacted with a compound of formula (XX) in which p has the same meaning as in formula (I) above, the value of p being equal of different to the value of n in the compound of formula (XIX), in the presence of potassium carbonate and potassium iodide. The resulting mixture is then heated at reflux for 8 to 24 hours to lead to the corresponding compound of formula (XXI) in which R⁵, R², m, n and p are as defined previously. The resulting compound of formula (XXI) can then be unprotected by addition of a strong acid, such as for example trifluoroacetic acid, into a solution of said compound of formula (XXI) in an appropriate solvent such as for example dichloromethane, and stirring at room temperature for 1 to 2 hours to lead to the expected corresponding compound of formula (I) which can then be recovered and purified according to the usual practice known from one skilled in the art.

Compounds of formula (XX) can be prepared according to a process comprising the step of reacting tosyl chloride with a compound of formula (XXII): OHCH₂CH₂-(OCH₂CH₂)ₚ₁-C(O)O-*t*-Butyl wherein p has the same meaning as in formula (I) above.

This process can be represented by the following Scheme 5:

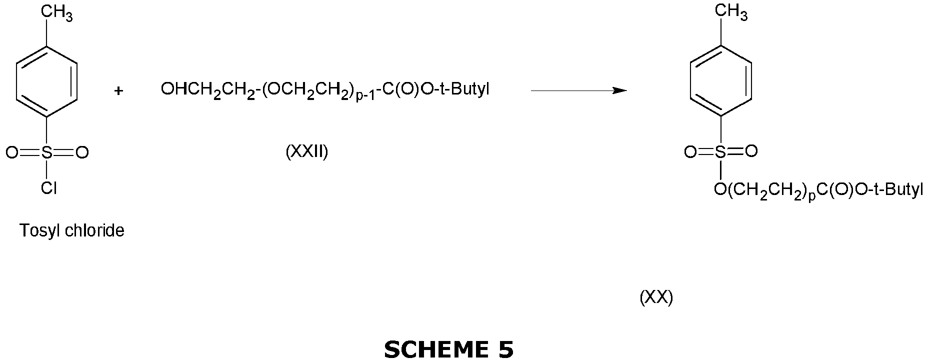

According to the process represented by Scheme 5, tosyl chloride is reacted with a solution of a compound of formula (XXII) in which p has the same meaning as in the compound of formula (I) and wherein p can have the same value than n or a different value than n, in an appropriate solvent such as for example dichloromethane, in the presence of an amine such as for example trimethylamine at room temperature for 8 to 24 hours. Compound of formula (XXII) thus obtained can then be recovered and purified by the technics well known by one skilled in the art.

The compounds of formula (I) in which R¹ is:
^{∗} an alkyl radical having at least 2 carbon atoms or an alkyl radical having at least 2 carbon atoms and comprising a terminal fluorinated group; or
^{∗} a group -OR⁴ or -COOR⁴ in which R⁴ represents a linear alkyl radical having at least 4 carbon atoms or an alkyl radical having at least 2 carbon atoms and comprising a terminal fluorinated group,
can be prepared according to a process comprising the step of reacting a compound of formula (II'):

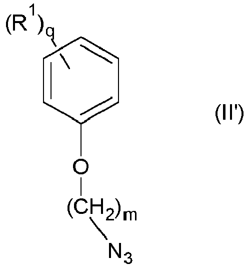

Wherein m is as defined in formula (I) and R¹ is an alkyl radical having at least 2 carbon atoms or an alkyl radical having at least 2 carbon atoms and comprising a terminal fluorinated group, or a group -OR⁴ or -COOR⁴ in which R⁴ represents a linear alkyl radical having at least 4 carbon atoms or an alkyl radical having at least 2 carbon atoms and comprising a terminal fluorinated group, and q is an integer ranging from 1 to 3, with a compound of formula (III) as described above when, in the desired compound of formula (I), n and p are identical or different and R² is identical to R³, or with a compound of formula (XVII) as described above when, in the desired compound of formula (I), n and p are identical or different and R² is an alkyloxy group as defined above in formula (I) and R³ is a carboxyl group.

The conditions previously described for reacting a compound of formula (II) with a compound of formula (III) or with a compound of formula (XVII) also apply respectively to the reaction of a compound of formula (II') with a compound of formula (III) or with a compound of formula (XVII).

Compounds of formula (II') which would not be commercially available can be prepared by analogy according to methods well known by one skilled in the art and described for example in "Spacing-dependent dipolar interactions in dendronized magnetic iron oxide nanoparticle 2D arrays and powders." Solenne Fleutot, et al., Nanoscale, 2013, 5 , 1507.

The dendritic molecules of formula (I) according to the present invention are useful as drug carriers, in particular as delivery vehicles of drugs, in particular of nonsteroidal anti-inflammatory drugs (NSAIDs), anticancer drugs, fragment antibodies, nanobodies, and other drugs such as simvastatin, famotidine, or quinolones.

Therefore, another object of the present invention is the use of a dendritic molecule of formula (I) as defined according to the first object of the present invention, as a drug carrier.

In particular, the dendritic molecule of formula (I) in which R³ is a carboxyl group are particularly preferred for a use as a drug carrier, since they can be easily functionalized with a ligand or an active principle.

The dendritic molecules of formula (I) according to the present invention are useful as contrast agents for medical imaging or diagnosis platforms after functionalization with at least one imaging agent such as for example biocompatible fluorescent dyes, traditional small molecular contrast agents, or metal ion/chelator complexes or with at least one metallic or metallic oxide nanoparticle. In particular, dendritic molecules of formula (I) can be grafted to metallic oxide nanoparticles and used as medical imaging tool, in particular optical imaging tool or magnetic imaging tool, more particularly magnetic resonance imaging contrast agent, or magnetic particle imaging tracer, or as a hyperthermia and/or radiosensitizing agent for the treatment of tumors or other pathological tissues.

Therefore, another object of the present invention is a dendritic molecule of formula (I) as defined according to the first object of the present invention in combination with at least one imaging agent, for its use as contrast agent for medical imaging or diagnosis platforms.

The dendritic molecules of formula (I) according to the invention are also particularly useful in phase change emulsions (PCEs) to (i) control the size and stabilize nanodroplets, (ii) precisely control the phase change phenomenon, (iii) obtain predetermined microbubbles sizes and size distributions, and (iv) stabilize these microbubbles.

The dendritic molecules of formula (I) according to the invention are also finally useful in controlling the properties of nanoemulsions and microbubbles, independently of the PCE.

Other advantages and embodiments of the present invention are given by the following examples.

### EXAMPLES

### EXAMPLE 1: Synthesis of a dendritic molecule of formula (I-A) according to the invention

In this example, a dendritic molecule of the following formula (I-A) was prepared:

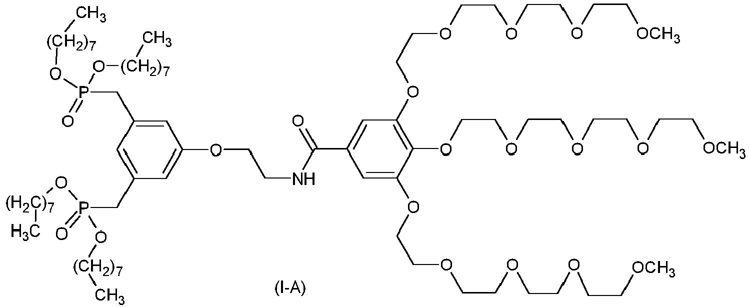

### 1.1 Step 1 - Preparation of 2,5,8,11-tetraoxatridecan-13-yl 4-methylbenzenesulfonate (compound 1)

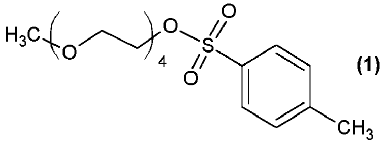

30.1 mmol (1 Eq.) of tetraethyleneglycol monomethyl ether were dissolved in 170.0 mL of dichloromethane at room temperature, then 5 mL of triethylamine (Et₃N) (36.2 mmol, 1.2 Eq.) were added and the reaction stirred for 10 min. 22.3 g (36.2 mmol, 1.2 Eq.) of tosyl chloride (solid) were slowly added. The resulting mixture was stirred at room temperature during the night.

A thin layer chromatography (TLC) analysis (stain phosphomolybdic acid (PMA) solution) showed the full consumption of PEG-OMe. The mixture was filtered on celite to remove the salts. The solvent was then evaporated under reduced pressure.

Purification by flash chromatography (100% dichloromethane (DCM) then 100% ethyl acetate (AcOEt)) afforded a clear liquid (10.1 g, 93%).

### Analysis:

¹H NMR (400 MHz, MeOD) δ 7.85 (d, J = 8.4 Hz, 2H), 7.50 (dd, J = 8.6, 0.8 Hz, 2H), 4.23 - 4.14 (m, 3H), 3.74 - 3.68 (m, 3H), 3.68 - 3.64 (m, 7H), 3.62 - 3.55 (m, 7H), 3.40 (s, 3H), 2.51 (s, 3H).

### 1.2 Step 2- Preparation of compound (2)

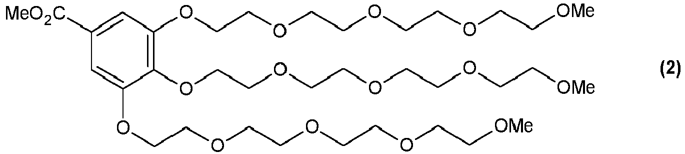

To a solution 8.8 mmol (1.0 Eq) of methyl gallate in acetone (60 mL) were added 18.1 mmol (3.2 Eq.) of potassium carbonate (K₂CO₃), 0.6 mmol (0.1 Eq.) of potassium iodide (KI) and 18.9 mmol (3.3 Eq.) of compound (1) as preparation in step 1 above. The resulting solution was heated to reflux for 42 hours.

The reaction mixture was cooled to room temperature, the solvent was removed, solid were suspended in dichloromethane (CH₂Cl₂), filtered over Celite, washed with an aqueous solution of sodium thiosulfate (Na₂S₂O₃) 2N and brine, dried over sodium sulfate (Na₂SO₄), filtered and concentrated under reduced pressure. Purification by flash chromatography (CH₂Cl₂/MeOH 98/2 to 96/4 to 9/1) gave 3.9g (89%) of a yellowish oil.

### Analysis:

¹H NMR (400 MHz, Chloroform-d) δ 7.46 (d, 2H: Ar-H), 4.15 (dd, J = 5.6, 4.2 Hz, 6H: -O-C*H*₂-R), 3.86 (s, 3H: C*H*₃-O-C=O-R), 3.85 - 3.81 (m, 6H: -O-CH₂-C*H*₂-R), 3.70 - 3.66 (m, 6H: -O-C*H*₂-R), 3.63 - 3.56 (m, 24H: -O-C*H*₂-R), 3.51 - 3.47 (m, 6H: -O-C*H*₂-R), 3.33 (s, 9H: C*H*₃-O-).

### 1.3 Step 3 - Preparation of compound (3)

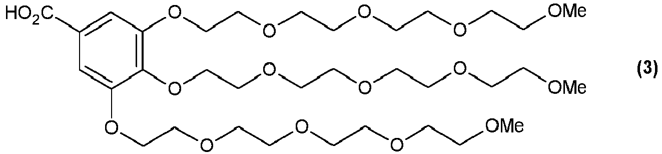

To a solution of 6.0 g (6.9 mmol - 1.0 Eq.) of compound (2) in methanol (MeOH) were successively added 1.1 g (34.5 mmol - 5.0 Eq.) of sodium hydroxide (NaOH) and 15.0 mL of distilled water. The yellow solution was stirred at room temperature (RT) overnight.

TLC analysis showed the consumption of compound (2). The solvents were removed by rotavap, then the crude product dissolved in CH₂Cl₂. HCl 2N (20.0 mL) was added and the stirring was continued for 15 min. The organic product was collected with CH₂Cl₂, the aqueous layer was washed with CH₂Cl₂ (five times), the combined organic layer was washed with brine, dried over Na₂SO₄, filtered and concentrated under reduced pressure to yield to compound (3) (5.1 g, 6.8 mmol, 99%) as yellow oil.

### Analysis:

¹H NMR (400 MHz, Chloroform-d) δ 7.46 (d, 2H : Ar-H), 4.15 (dd, J = 5.6, 4.2 Hz, 6H: -O-C*H*₂-R), 3.85 - 3.81 (m, 6H : -O-CH₂-C*H*₂-R), 3.70 - 3.66 (m, 6H : -O-C*H*₂-R), 3.63 - 3.56 (m, 24H : -O-C*H*₂-R), 3.51 - 3.47 (m, 6H : -O-C*H*₂-R), 3.33 (s, 9H : *CH3-O-).*

### 1.4 Step 4 - Preparation of 3,5-bis(bromomethyl)phenol (compound (4))

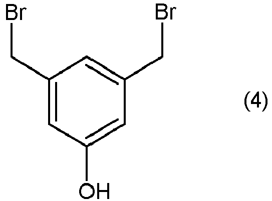

72 mL of lithium aluminum hydride 1M (LiAlH₄) (72 mmol - 1.8 Eq.) were dissolved in 150 mL of tetrahydrofuran (THF) at 0°C. Then 8.40 g (40 mmol - 1 Eq.) of dimethyl 5-hydroxyisophthalate were carefully added. The resulting solution was stirred at RT for 5 hours, then EtOAc (30.0 mL) and an aqueous solution of H₂SO₄ 10% (60.0 mL) were carefully added at 0°C. The stirring was continued overnight. An additional aqueous solution of H₂SO₄ 10% (60.0 mL) was added. The stirring was continued at RT for another 24h.

Once the aluminum salt was entirely dissolved, the aqueous layer was washed with EtOAc (at least 5 times). The combined organic layer was dried over Na₂SO₄, filtered and concentrated under reduced pressure to yield 8.3831 g of orange oil. The crude benzylic alcohol was then dissolved in 100 mL of acetic acid (AcOH) before 36 mL of hydrobromic acid (HBr 33% w/w in AcOH) (200 mmol - 5.0 Eq.) was carefully added at 0°C. The resulting mixture was stirred at RT for 2 days.

TLC analysis showed the full consumption of bis-benzylic alcohol, which is the intermediate formed but not isolated following the reduction of dimethyl 5-hydroxyisophthalate by LiAlH₄. The organic product was washed with brine (1 time), the aqueous layer was washed with EtOAc (at least five times), the combined organic layer was dried over Na₂SO₄, filtered and concentrated under reduced pressure. Purification by flash chromatography (CH₂Cl₂/EtOAc 1/0 to 96/4 to 9/1) afforded a yellowish solid. Recrystallization with a mixture of petroleum ether/diethyl oxide (EtP/Et₂O) afforded compound (4) (10.40 g, 37.4 mmol, 93.6%) as white solid.

### Analysis:

¹H NMR (400 MHz, Chloroform-d) δ 6.99 (s, 1H: Ar-H), 6.81 (d, J = 1.43 Hz, 2H: Ar-H), 4.41 (s, 4H: -C*H*₂-Ar).

### 1.5. Step 5 - Preparation of compound (5)

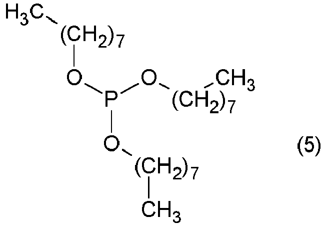

In a 100 mL two-necked flask connected to a Dean-Stark, 33.9 mL of octanol (107.25 mmol - 6 Eq.) were added, then the reaction setup was stirred and passed under a flow of argon for 10 min.

After that, the liquid was heated to 75°C and the flow replaced by 2 balloons of argon. 4.23 mL of trimethylphosphite (35.8 mmol - 1 Eq.) were added slowly from the syringe and then the setup was heated at 220°C for 6 h.

Then the setup was cooled to ambient temperature, and the Dean-Stark containing the methanol formed during the reaction removed. An orange oil was obtained.

Distillation under reduced pressure was used to separate the remaining octanol from the compound (5) formed, at 160°C.
9.205 g of orange oil were recovered (yield = 61%)

### Analysis:

¹H-NMR (400MHz, Chloroform-d): δ 3.77 (q, J = 6.9 Hz, 6H, O-C*H*₂-R), 1.61 - 1.57 (m, 6H, O-C*H*₂-CH₂-R), 1.28 - 1.25 (m, 30H, C*H*₂), 0.86 (t, J = 6.7 Hz, 9H, C*H*₃-R).
31P-NMR (400MHz, CDCl₃): δ 139.15

### 1.6. Step 6 - Preparation of compound (6)

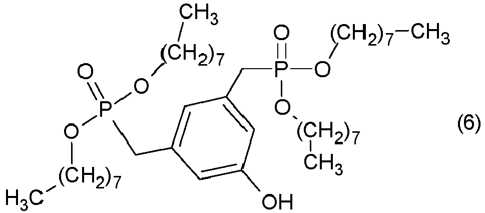

152 mg (0.543 mmol - 1 Eq.) of compound (4) and 916 mg (2.188 mmol - 4.0 Eq.) of compound (5) were added to a 50 mL flask. The reaction was stirred and heated to 140°C. overnight. A yellow-orange oil was obtained and a TLC was carried out (Petroleum ether / EtOAc 1/1):
The next day, after having cooled the solution, a Petroleum Ether / EtOAc chromatographic column was produced (1/1 to 2/3).

248 mg of compound (6) were recovered (yield = 64%).

### Analysis:

¹H NMR (400 MHz, CDCl₃) δ 6.81 (s, 2H, Ar-H), 6.64 (s, 1H, Ar-H), 4.02 - 3.77 (m, 8H, P-C*H*₂), 3.28 - 2.81 (m, 4H, Ar-C*H*₂-P), 1.58 (m, 8H, P-CH₂-C*H*₂), 1.42 - 1.14 (m, 44H, C-C*H*₂), 0.95 - 0.77 (t, J = 7.1Hz, 12H, C-C*H*₃).

³¹P NMR (162 MHz, CDCl₃) δ 26.51.

### 1.7. Step 7 - Preparation of linker 2 (L2)

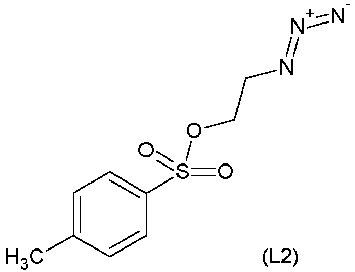

### 1.7.1. Step 7.1 - Preparation of linker 1 (L1)

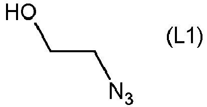

10.0 g (77.6 mmol - 1 Eq.) of 2-bromoethanol (97%) were dissolved in 12 mL of water, then at RT 6.12 g (93.2 mmol - 1.2 Eq.) of sodium azide (NaN₃) were added. The mixture was heated at 80°C for the night. Monitoring was made by TLC (DCM/MeOH, 95:5, SM Rf=0, EP Rf= 0,5).

After the night TLC showed just a few traces of the starting material, the mixture was stopped, then 10 mL of DCM was added. Phases were separated. Aqueous was extracted with 20 mL of DCM and NaCl solid was added at each extraction. The organic phase was directly engaged in the next step, without evaporation or further purification.

### 1.7.2. Step 7. - Preparation of linker 2 (L2)

To 6.76 g (77.6 mmol - 1 Eq.) of Linker 1 in DCM, were added 16.4 mL (116.0 mmol - 1.5 Eq.) of Et₃N slowly, the mixture was stirred 10 min, then 17.9 g (93.2 mmol - 1.2 Eq.) of tosyl chloride (TsCI) were added slowly.

After the night TLC (petroleum ether, AcOEt 7:3, KMnO₄) showed traces of the starting material. The mixture was concentrated, TsCI salts were precipitated in AcOEt and filtered over celite pad, washed 3 times with AcOEt, to give the desired linker 2 (22 g, brown oil).

It was then purified by Flash chromatography, Interchim-220g-30, liquid deposit in EtP/AcOEt (9:1), Eluent: 15 min EtP/AcOEt (9:1), 10 min EtP/AcOEt (8:2) with isocratic, and 10min EtP/AcOEt (7:3).
12.13 g of Linker 2 as a colourless oil was recovered.
¹H NMR (400 MHz, MeOD) δ 7.84 (d, J = 8.4 Hz, 2H), 7.49 (d, J = 7.8 Hz, 2H), 4.25 - 4.12 (m, 2H), 3.54 - 3.40 (m, 3H), 2.49 (s, 3H).

### 1.8. Step 8 - Preparation of compound (7)

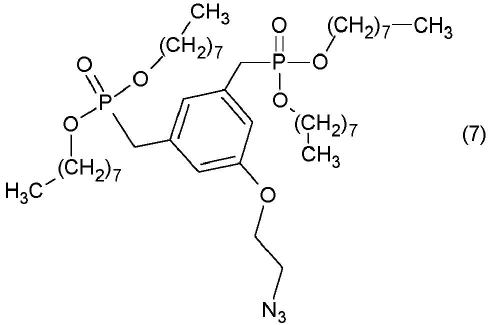

In a 25 mL flask were added 297.0 mg (1.23 mmol - 1.8 Eq.) of compound (6), 57.2 mg (1.02 mmol - 1.5 Eq.) of potassium hydroxide (KOH) and 11.3 mg (0.068 mmol - 0.1 Eq.) of KI in 15 mL of toluene. The solution was stirred for 20 min and heated to 60°C. to dissolve the reagents. 500.0 mg (0.684 mmol - 1.0 Eq.) of linker 2 (as prepared above in step 1.7. of example 1) was added and the reaction was left to stir overnight.

The flask was cooled to RT, then the toluene was evaporated off under reduced pressure. The crude as then dissolved in DCM, and filtered through Celite. A chromatographic column was carried out DCM / MeOH (1/0 to 98/2 to 96/4). 530 mg of compound (7) (97%) were thus recovered.

### Analysis:

¹H NMR (400 MHz, MeOD) δ 6.90 - 6.82 (m, 3H, Ar-H), 4.17 (t, J = 4.9 Hz, 2H, O-C*H*₂-CH₂-N₃), 3.98 (m, 8H, P-O-C*H*₂), 3.59 (t, J = 4.8 Hz, 2H, O-CH₂-C*H*₂-N₃), 3.27 - 3.16 (BX, J = 21.9 Hz, 4H, Ar-C*H*₂-P), 1.67 - 1.55 (m, 8H, P-O-CH₂-C*H*₂), 1.41 - 1.26 (m, 40H, C-C*H*₂), 0.95 - 0.85 (t, J = 6.5Hz, 12H, C-C*H*₃).

¹³C NMR (101 MHz, MeOD) δ 160.00, 134.57, 134.51, 134.45, 125.49, 115.95 *(C-Ar),* 68.46, 67.76, 67.73, 67.69, 51.32, 34.29, 33.01, 32.92, 31.67, 31.64, 31.61, 30.41, 30.29, 26.70, 23.75, 14.47 (C-CH₃).

³¹P NMR (162 MHz, MeOD) δ 27.11.

### 1.9. Step 9 - Preparation of a dendritic molecule of formula (I-A)

To a solution of 350.0 mg (0.434 mmol - 1.1 Eq.) of compound **(7)** in 15 mL of EtOAc were added 120 mg (0.1 Eq.) of Pd/C 10% as catalyst. The resulting mixture was purged with an atmosphere of hydrogen (five times), then vigorously stirred at RT for 5h. In a separate flask, 300 mg (0.398 mmol - 1 Eq.) of compound **(3)** were dissolved in 10.0 mL of CH₂Cl₂ before 0.22 mL (1.2 mmol - 3.0 Eq.) of oxalyl chloride (COCl)₂ and 4 drops of dimethyl formamide (DMF) were added. The orange solution was stirred at RT for 5h. When TLC confirmed the full consumption of compound **(7),** the catalyst was filtered over Celite and the crude product was concentrated under reduced pressure at RT. The acyl chloride was simultaneously concentrated under reduced pressure, dissolved in 10 mL of CH₂Cl₂ then the crude primary amine (intermediate product formed but not purified) and 0.16 mL (0.916 mmol - 2.3 Eq.) of N,N-Diisopropylethylamine (DIPEA) were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure to yield 7.17 g orange oil. Purification by flash chromatography (100% EtOAc then CH₂Cl₂/EtOAc/MeOH 80/12/8 to 6/0/4) were conducted to separate the pure and impure fractions, to afford the dendritic molecule of formula **(I-A)** (487.2 mg, 81%) as a colorless oil.

### Analysis:

¹H NMR (400 MHz, MeOD) δ 7.23 (s, 2H), 6.85 (m Hz, 3H), 4.22 (m, 4H), 4.17 (t, J = 5.7 Hz, 2H), 3.97 (m, 8H), 3.91 - 3.84 (t, J = 4.6 Hz, 2H), 3.83 - 3.76 (m, 2H), 3.78 - 3.68 (m, 6H), 3.69 - 3.57 (m, 20H), 3.56 - 3.48 (m, 6H), 3.33 (m, 9H), 3.24 - 3.14 (ABx, J = 22 Hz, 4H), 1.60 (m, 8H), 1.29 (m, 38H), 0.90 (t, J = 6.8 Hz, 12H).

³¹P NMR (162 MHz, CDCl₃) δ 25.88.

### EXAMPLE 2: Synthesis of a dendritic molecule of formula (I-B) according to the invention

In this example, a dendritic molecule of the following formula **(I-B)** was prepared:

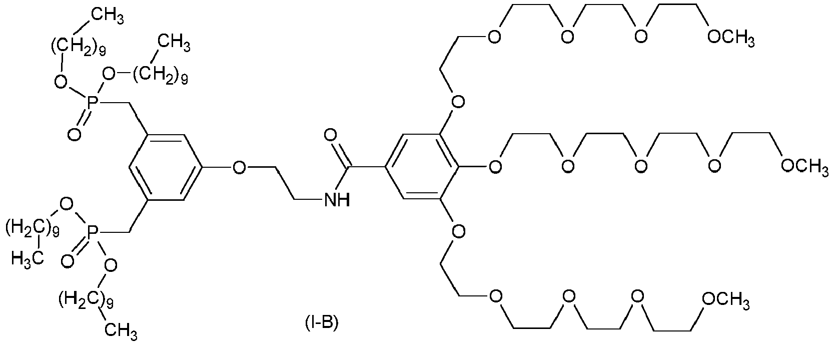

### 2.1. Step 1 - Preparation of compound (8)

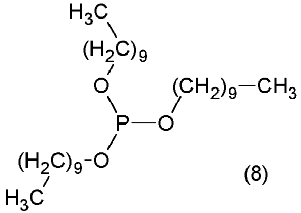

In a 100mL two-necked flask connected to a Dean-Stark, 91.4 mL (469.0 mmol - 6.0 Eq.) of decanol was added, then the reaction setup was stirred and passed under a flow of argon for 10 min.

After that, the liquid was heated to 75°C and the flow replaced by 2 balloons of argon. 9.51 mL (78.2 mmol - 1.0 Eq.) of trimethylphosphite were added slowly from the syringe and then the setup was heated at 220°C for 6 h.

Then the setup was cooled to ambient temperature, and the Dean-Stark containing the methanol formed during the reaction removed. An orange oil was obtained.

Distillation under reduced pressure was used to separate the remaining decanol from the compound (8) thus formed, at 160°C.

36.2 g of compound (8) as a colourless oil were recovered (yield = 92%).

### Analysis:

¹H NMR (400 MHz, CDCl₃) δ 3.77 (m, 6H), 1.64 - 1.55 (m, 6H), 1.41 - 1.18 (m, 44 H), 0.87 (t, J = 6.8 Hz, 9H).

¹³C NMR (101 MHz, CDCl₃) δ 62.48, 62.37, 32.04, 32.02, 31.33, 31.28, 29.76, 29.75, 29.71, 29.67, 29.64, 29.47, 29.43, 26.00, 22.82, 14.23.

31P-NMR (400MHz, CDCl₃): δ 139.19.

### 2.2. Step 2 - Preparation of compound (9)

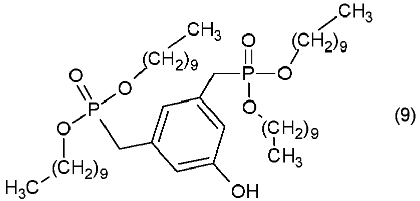

2.0 g (7.14 mmol - 1.0 Eq.) of compound **(4)** as prepared in step 1.4 of Example 1 above and 14.4 g (28.6 mmol - 4.0 Eq.) of compound **(8)** were added to a 100 mL flask. The reaction was stirred and heated to 140°C. overnight. A yellow-orange oil was obtained; a TLC was carried out (Petroleum ether / EtOAc 1/1).

The next day, after having cooled the solution, a Petroleum Ether / EtOAc chromatographic column was produced (1/1 to 2/3).

4.9 g of compound **(9)** were recovered (yield = 81 %).

### Analysis:

¹H NMR (400 MHz, CDCl₃) δ 6.81 - 6.77 (m, 2H), 6.67 (s, 1H), 3.92 (m, 8H), 3.11 - 3.01 (m, 4H), 1.67 - 1.51 (m, 8H), 1.26 (m, 56H), 0.94 - 0.82 (t, *J* = 6.9 Hz, 12H).
³¹P NMR (162 MHz, CDCl₃) δ 26.40
HRMS: m/z theo: C₄₈H₉₂O₇P₂ 842.63 g.mol⁻¹, measured: C₄₈H₉₂O₇P₂Na: 866.235 g.mol⁻¹

### 2.3. Step 3 - Preparation of compound (10)

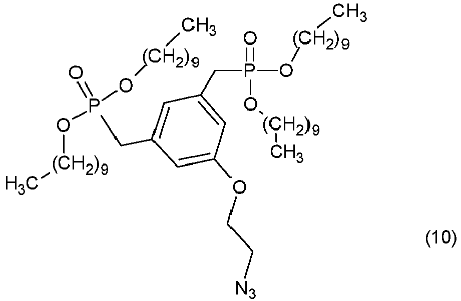

In a 100 mL flask were added 2.45 g (2.91 mmol - 1.0 Eq.) of compound **(9),** 0.25 g (4.36 mmol - 1.5 Eq.) of KOH and 49 g (0.3 mmol - 0.1 Eq.) of KI in 50 mL of toluene. The solution was stirred for 20 min and heated to 60°C. to dissolve the reagents. 1.05 g (4.36 mmol - 1.5 Eq.) of Linker 2 (as prepared above in step 1.7. of example 1) were added and the reaction was left to stir overnight.

The flask was cooled to room temperature, then the toluene was evaporated off under reduced pressure. The crude was then dissolved in DCM, and filtered through Celite. A chromatographic column was carried out DCM / MeOH (1/0 to 98/2 to 96/4). 2.1 g of compound **(10)** (79%) were thus recovered.

### Analysis:

¹H NMR (400 MHz, MeOD) δ 6.90 - 6.82 (m, 3H), 4.17 (t, J = 4.8 Hz, 2H), 3.98 (m, 8H), 3.63 - 3.56 (t, J = 4.9 Hz, 2H), 3.25 - 3.13 (d, J = 22 Hz, 4H), 1.62 (p, J = 6.6 Hz, 8H), 1.32 (m, 54H), 0.96 - 0.85 (t, J = 6.5 Hz, 12H).

¹³C NMR (101 MHz, MeOD) δ 160.00, 134.57, 134.45, 125.47, 115.96, 68.46, 67.77, 67.73, 67.70, 51.32, 34.31, 33.12, 32.94, 31.67, 31.64, 31.61, 30.75, 30.74, 30.51, 30.33, 26.71, 23.78, 14.48.

³¹P NMR (162 MHz, MeOD) δ 31.03.

### 2.4. Step 4 - Preparation of a dendritic molecule of formula (I-B)

To a solution of 237 mg (0.259 mmol - 1.1 Eq.) of compound **(10)** in 15 mL of EtOAc and 251 mg (0.1 Eq.) of Pd/C 10% as catalyst. The resulting mixture was purged with an atmosphere of hydrogen (five times), then vigorously stirred at RT for 5h. In a separate flask, 175 mg (0.236 mmol - 1.0 Eq.) of compound **(3)** as prepared above at step 1.3. of example 1 were dissolved in 15 mL of CH₂Cl₂ before 0.13 mL (0.71 mmol - 3.0 Eq.) of (COCl)₂ and 4 drops of DMF were added. The orange solution was stirred at RT for 5h. When TLC confirmed the full consumption of compound **(3),** the catalyst was filtered over Celite and the crude product was concentrated under reduced pressure at RT. The acyl chloride was simultaneously concentrated under reduced pressure, dissolved in CH₂Cl₂ then the crude primary amine and 0.11 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure to yield to an orange oil. Purification by flash chromatography (100% EtOAc then CH₂Cl₂/EtOAc/MeOH 80/12/8 to 6/0/4) were conducted to separate the pure and impure fractions, to afford the dendritic molecule of formula **(I-B)** (293 mg, 67%) as colorless oil.

### Analysis:

¹H NMR (400 MHz, MeOD) δ 7.23 (s, 2H), 6.85 (m, 2H), 4.26 - 4.13 (m, 6H), 4.08 - 3.90 (m, 8H), 3.88 - 3.85 (t, J = 4.2 Hz, 2H), 3.81 - 3.78 (m, 1H), 3.76 (t, J = 5.7 Hz, 1H), 3.71 (m, 4H), 3.67 - 3.57 (m, 19H), 3.55 - 3.49 (m, 4H), 3.37 - 3.31 (bs, 9H), 3.24 - 3.14 (ABₓ, J = 21.9 Hz, 4H), 1.67 - 1.55 (m, 8H), 1.29 (m, 55H), 0.90 (t, J = 6.9Hz, 12H).

³¹P NMR (162 MHz, MeOD) δ 27.14.

### EXAMPLE 3: Synthesis of a dendritic molecule of formula (I-C) according to the invention

In this example, a dendritic molecule of the following formula (I-C) was prepared:

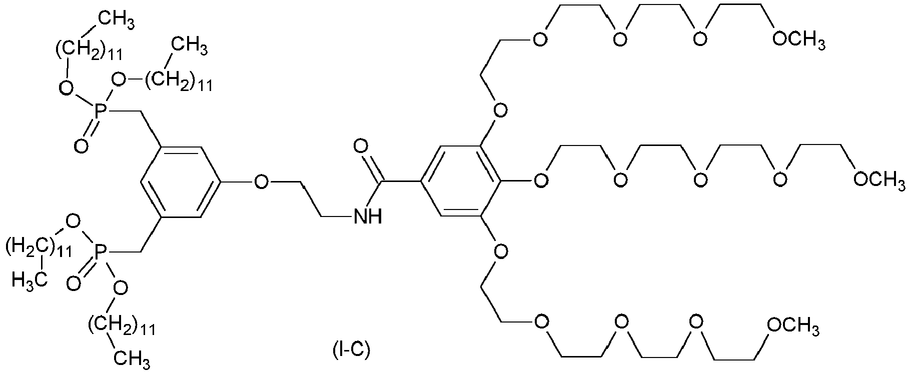

### 3.1. Step 1 - Preparation of compound (11)

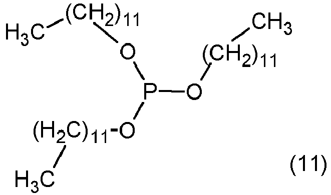

In a 250 mL two-necked flask connected to a Dean-Stark, 107.0 mL (469.0 mmol - 6.0 Eq.) of dodecanol was added, then the reaction setup was stirred and passed under a flow of argon for 10 min.

After that, the liquid was heated to 75°C and the flow replaced by 2 balloons of argon. 9.51 mL (78.2 mmol - 1.0 Eq.) of trimethylphosphite were added slowly from the syringe and then the setup was heated at 220°C for 6 h.

Then the setup was cooled to ambient temperature, and the Dean-Stark containing the methanol formed during the reaction removed. An orange oil was obtained.

Distillation under reduced pressure was used to separate the remaining dodecanol from the compound **(11)** thus formed, at 160°C.

43.0 g of compound **(11)** as a colourless oil were recovered (yield = 94%).

### Analysis:

¹H NMR (400 MHz, CDCl₃) δ 3.78 (m, 6H), 1.64 - 1.51 (m, 6H), 1.26 (m, 65H), 0.91 - 0.83 (t, J = 6.5 Hz, 9H).

¹³C NMR (101 MHz, CDCl₃) δ 62.97, 62.94, 62.44, 62.33, 32.88, 32.03, 31.29, 31.24, 29.79, 29.76, 29.73, 29.72, 29.58, 29.47, 29.44, 25.96, 25.89, 22.79, 14.17.

³¹P NMR (162 MHz, CDCl₃) δ 139.19.

### 3.2. Step 2 - Preparation of compound (12)

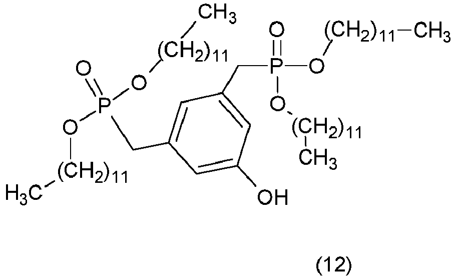

1.0 g (3.57 mmol - 1.0 Eq.) of compound **(4)** as prepared in step 1.4 of Example 1 above and 8.39 g (14.3 mmol - 4.0 Eq.) of compound **(11)** were added to a 100 mL flask. The reaction was stirred and heated to 140°C. overnight. A yellow-orange oil was obtained; a TLC was carried out (Petroleum ether / EtOAc 1/1).

The next day, after having cooled the solution, a Petroleum Ether / EtOAc chromatographic column was produced (1/1 to 2/3).

2.28 g of compound **(12)** were recovered (yield = 67 %).

### Analysis:

¹H NMR (400 MHz, CDCl₃) δ 6.80 (s, 2H), 6.66 (s, 1H), 3.92 (dq, J = 13.0, 8.0 Hz, 8H), 3.11 - 3.01 (m, 4H), 1.58 (t, J = 6.6 Hz, 8H), 1.25 (s, 75H), 0.88 (t, J = 6.9 Hz, 12H).

¹³C NMR (101 MHz, CDCl₃) δ 157.61, 132.68, 122.76, 115.79 (C, Ar), 66.45 (CH₂-O-R), 31.94, 30.61, 29.70, 29.68, 29.64, 29.59, 29.38, 29.25, 25.53, 23.84, 22.70 (*C*H₂), 14.12 (*C*H₃).

³¹P NMR (162 MHz, CDCl₃) δ 26.40.

C₄₈H₉₂NaO₇P₂ m/z calculated: 978,3898, m/Z found: 978,3990.

### 3.3. Step 3 - Preparation of compound (13)

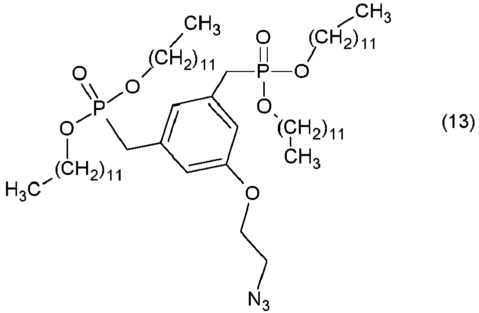

In a 100 mL flask were added 500 mg (0.523 mmol - 1.0 Eq.) of compound **(12),** 44.0 mg (0.785 mmol - 1.5 Eq.) of KOH and 26 mg (0.157 mmol - 0.1 Eq.) of KI in 25 mL of toluene. The solution was stirred for 20 min and heated to 60°C. to dissolve the reagents. 252.4 mg (0.785 mmol - 1.5 Eq.) of Linker 2 (as prepared above in step 1.7. of example 1) were added and the reaction was left to stir overnight.

The flask was cooled to room temperature, then the toluene was evaporated off under reduced pressure. The crude was then dissolved in DCM, and filtered through Celite. A chromatographic column was carried out DCM / MeOH (1/0 to 98/2 to 96/4). 500 mg of compound **(13)** (93%) were thus recovered.

### Analysis:

¹H NMR (400 MHz, CDCl₃) δ 6.87 - 6.68 (m, 3H), 4.13 (t, J = 5.0 Hz, 2H), 4.01 - 3.85 (m, 8H), 3.56 (d, J = 5.1 Hz, 2H), 3.08 (ABx, J = 21.9 Hz, 4H), 1.64 - 1.52 (m, 8H), 1.32 - 1.20 (m, 72H), 0.88 (t, J = 6.6 Hz, 12H).

³¹P NMR (162 MHz, CDCl₃) δ 25.94.

### 3.4. Step 4 - Preparation of a dendritic molecule of formula (I-C)

To a solution of 305 mg (0.297 mmol - 1.1 Eq.) of compound **(13)** in 15 mL of EtOAc were added 320 mg (0.1 Eq.) of Pd/C 10% as catalyst. The resulting mixture was purged with an atmosphere of hydrogen (five times), then vigorously stirred at RT for 5h. In a separate flask, 200 mg (0.270 mmol - 1.0 Eq.) of compound **(3)** as prepared above at step 1.3. of example 1 were dissolved in 15 mL of CH₂Cl₂ before 0.15 mL (0.81 mmol - 3.0 Eq.) of (COCl)₂ and 4 drops of DMF were added. The orange solution was stirred at RT for 5h. When TLC confirmed the full consumption of compound **(3),** the catalyst was filtered over Celite and the crude product was concentrated under reduced pressure at RT. The acyl chloride was simultaneously concentrated under reduced pressure, dissolved in CH₂Cl₂ then the crude primary amine and 0.11 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced Purification by flash chromatography (100% EtOAc then CH₂Cl₂/EtOAc/MeOH 80/12/8 to 6/0/4) were conducted to separate the pure and impure fractions, to afford the dendritic molecule of formula **(I-C)** (265 mg, 58%) as colorless oil.

### Analysis:

¹H NMR (400 MHz, CDCl₃) δ 7.12 (s, 2H), 6.74 (m, 3H), 4.19 (m, 4H), 4.09 (t, J = 5.8 Hz, 2H), 3.90 (m, 8H), 3.82 (t, J = 4.8 Hz, 2H), 3.78 - 3.71 (m, 4H), 3.61 (m, 19H), 3.51 - 3.48 (m, 4H), 3.35 - 3.27 (m, 9H), 3.10 - 2.97 (ABx, J = 21.8 Hz, 4H), 1.55 (h, J = 6.2 Hz, 8H), 1.22 (m, 76H), 0.84 (t, J = 6.4 Hz, 12H).

³¹P NMR (162 MHz, CDCl₃) δ 25.88.

### EXAMPLE 4: Synthesis of a dendritic molecule of formula (I-D) according to the invention

In this example, a dendritic molecule of the following formula **(I-D)** was prepared:

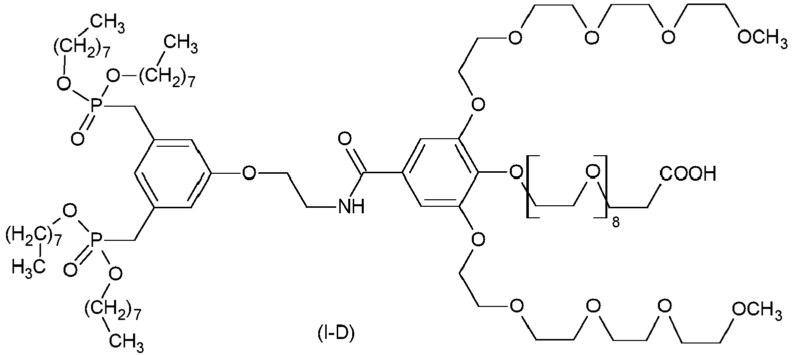

### 4.1. Step 1 - Preparation of compound (14)

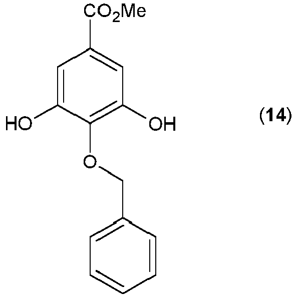

To a solution of 30 g (163 mmol - 1 Eq.) of methyl gallate in 150 mL of DMF were successively added 49.1 g (489 mmol - 4.5 Eq.) of KHCO₃, 0.136 g (0.82 mmol - 0.006 Eq.) of KI and 21.3 mL (163 mmol - 1.0 Eq.) of benzyl bromide (BnBr). The resulting mixture was stirred at RT for 36h.

The solids were filtered over Celite, the filtrate was acidified with an aqueous solution of HCl 2N (50.0 mL). The aqueous mixture was extracted with EtOAc (three times), the combined organic layer was washed with an aqueous saturated solution of NaHCO₃ (two times), brine (three times), dried over Na₂SO₄, filtered, concentrated under reduced pressure and dried under vacuum overnight. Purification with a chromatographic column (DCM/MeOH, 100:0 to 98:2 to 96:4 to 90:10) gave a translucent oil. The mixture was then dissolved with a small amount of AcOEt and Petroleum ether was poured slowly until precipitation occurred. Compound **(14)** was obtained as a white solid (16.5g, 38%)

### Analysis:

¹H NMR (400 MHz, MeOD) δ 7.53 - 7.44 (m, 2H, Ar-H), 7.30 (qd, J = 6.9, 3.7 Hz, 3H, Ar-H), 7.01 (d, J = 1.3 Hz, 2H, Ar-H), 5.14 (s, 2H, Ar-C*H*₂), 3.82 (d, J = 1.5 Hz, 3H, O-C*H*₃).

¹³C NMR (101 MHz, MeOD) δ 168.49, 151.90, 139.62, 138.80, 129.87, 129.19, 129.16, 129.11, 126.44 (12C, C-Ar), 110.07 (1C, C-COOR), 75.12 (1C, Ar-CH₂), 52.46 (1C, O-*C*H₃).

### 4.2. Step 2 - Preparation of compound (15)

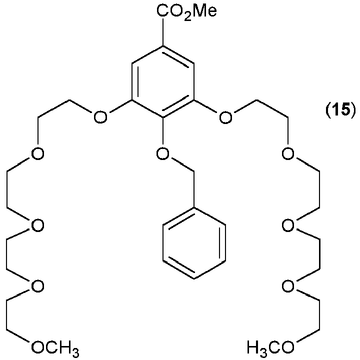

To a solution of 5.81 g (16.1 mmol - 2.2 Eq.) of compound **(14)** in 150.0 mL of acetone were added 3.22 g (23.4 mmol - 3.2 Eq.) of K₂CO₃,0.12 g (0.73 mmol - 0.4 Eq.) of KI and 2.0 g (7.3 mmol - 1.0 Eq.) of compound **(1)** as prepared in step 1.1 of example 1. The resulting solution was heated to reflux overnight.

The reaction mixture was cooled to RT, the solvent was removed, solids were suspended in CH₂Cl₂, filtered over Celite, washed with an aqueous solution of Na₂S₂O₃ 2N and brine, dried over Na₂SO₄, filtered and concentrated under reduced pressure. Purification by flash chromatography (CH₂Cl₂/MeOH 98/2 to 96/4 to 9/1) gave 3.3 g (71%) of compound **(15)** in the form of a yellowish oil.

### Analysis:

¹H NMR (400 MHz, Chloroform-d) δ 7.46 (d, 2H : Ar-H), 7.35 - 7.20 (m, 5H : Ar-H), 5.09 (s, 2H : -C*H*₂-OAr), 4.15 (dd, J = 5.6, 4.2 Hz, 4H: -O-C*H*₂-R), 3.86 (s, 3H : C*H*₃-O-C=O-R), 3.85 - 3.81 (m, 4H : -O-CH₂-C*H*₂-R), 3.70 - 3.66 (m, 4H : -O-C*H*₂-R), 3.63 - 3.56 (m, 16H : -O-C*H*₂-R), 3.51 - 3.47 (m, 4H : -O-C*H*₂-R), 3.33 (s, 6H : C*H₃*-O-).

¹³C NMR (101 MHz, CDCl₃) δ 169.26, 153.95, 142.97, 139.17, 129.74, 129.22, 129.01, 127.17, 109.67, 75.95, 72.93, 71.83, 71.65, 71.57, 71.50, 71.32, 70.81, 69.98, 59.06.

### 4.3. Step 3 - Preparation of compound (16)

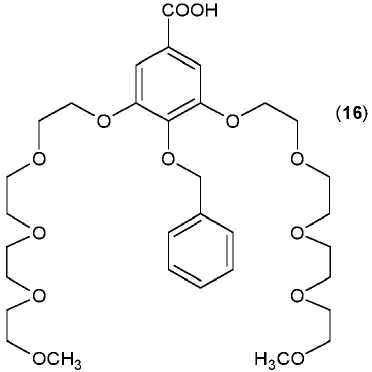

1.01 g (25.22 mmol - 5.0 Eq.) of NaOH were added to a solution of 3.3 g (5.04 mmol - 1.0 Eq.) of compound **(15)** in 27 mL of MeOH and 3 mL of distilled water. The orange solution was stirred at RT overnight.

The solvent was removed, the crude product was suspended in CH₂Cl₂, quenched with an aqueous solution of HCl 2N, the aqueous layer was extracted with CH₂Cl₂ (five times), the combined organic layer was dried over Na₂SO₄, filtered and concentrated under reduced pressure to yield to compound **(16)** (3.05 g, 95%) as a light yellow oil.

### Analysis:

¹H NMR (400 MHz, Chloroform-d) δ 7.46 (d, 2H : Ar-H), 7.35 - 7.20 (m, 5H : Ar-H), 5.09 (s, 2H : -C*H*₂-OAr), 4.15 (dd, J = 5.6, 4.2 Hz, 4H: -O-C*H*₂-R), 3.85 - 3.81 (m, 4H : -O-CH₂-C*H*₂-R), 3.70 - 3.66 (m, 4H : -O-*CH₂*-R), 3.63 - 3.56 (m, 16H : -O-C*H*₂-R), 3.51 - 3.47 (m, 4H : -O-C*H*₂-R), 3.33 (s, 6H : *CH3-O-).*

### 4.4. Step 4 - Preparation of compound (17)

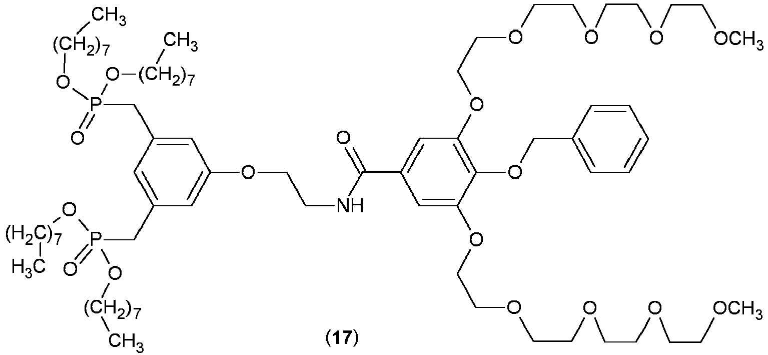

To a solution of 1.67 g (2.09 mmol - 1.0 Eq.) of compound **(7)** as prepared at step 1.8 of example 1 in 40 mL of EtOAc were added 1.1 g (0.1 Eq.) of Pd/C 10% as catalyst. The resulting mixture was purged with an atmosphere of hydrogen (five times), then vigorously stirred at RT for 5h. When TLC confirmed the full consumption of compound **(7),** the catalyst was filtered over Celite and the crude product was concentrated under reduced pressure at RT. In parallel, compound **(16)** (1.00 eq., 2.09 mmol, 1.34 g) was dissolved in DCM (67.0 mL), then 1-ethyl-3-(3-dimethylaminopropyl)carbodiimide (EDC) (1.50 eq., 3.13 mmol, 612 mg), and DMAP (0.10 eq., 0.21 mmol, 26 mg) were added, and the reaction stirred for 10 min before adding the crude primary amine. The resulting mixture was stirred at RT overnight. Monitoring by TLC DCM/MeOH, 6:4, KMnO4. The reaction mixture was concentrated under reduced pressure, the crude was purified by flash chromatography (SiO₂ 40.0 g, solid deposit in DCM, eluent: DCM/MeOH 100/0 to 90/10) and give expected product **(17)** (2.55 g, 89% yield) as a colorless oil

### Analysis:

1H NMR (400 MHz, MeOD) :δ 7.54 - 7.52 (m, 2H, Ar-H), 7.36 - 7.28 (m, 3H, Ar-H), 7.21 (s, 2H, Ar-H), 6.86 - 6.85 (m, 3H, Ar-H), 5.49 (s, 3H, ?), 5.10 (s, 2H, O-C*H*₂-Ar), 4.19 (t, 4H, Ar-O-C*H*₂-CH₂-O, J), 4.18 (t, 2H, O-C*H₂*-CH₂-N, J), 3.99 - 3.94 (m, 9H, P-O-CH₂-CH₂), 3.88 - 3.86 (m, 4H, Ar-O-CH₂-CH₂-O), 3.77 - 3.74 (t, 2H, O-CH₂-C*H*₂-N, J), 3.72 - 3.69 (m, 4H, O-CH₂-C*H*₂-O), 3.63 - 3.54 (m, 17H, O-CH₂-C*H*₂-O), 3.49 - 3.47 (m, 4H, ...), 3.31 (s, 6H, O-C*H*₃), 3.19 (d, 4H, P-C*H*₂, J), 1.62 - 1.58 (m, 9H, P-O-CH₂-CH₂-), 1.33 - 1.28 (m, 44H), 0.89 (t, 12H, CH₂-CH3, J).

¹³C NMR (101 MHz, MeOD) δ 169.56, 160.38, 154.04, 141.70, 139.20, 130.67, 129.73, 129.22, 129.00, 115.98, 107.49, 75.94, 72.93, 71.83, 71.64, 71.56, 71.51, 71.32, 70.82, 69.99, 67.74, 67.71, 67.67, 67.54, 59.09, 54.80, 34.29, 32.98, 31.65, 31.62, 31.59, 30.69, 30.38, 30.26, 26.68, 23.73, 14.49.

³¹P NMR (400 MHz, Methano-d4): δ 27.16

### 4.5. Step 5 - Preparation of compound (18)

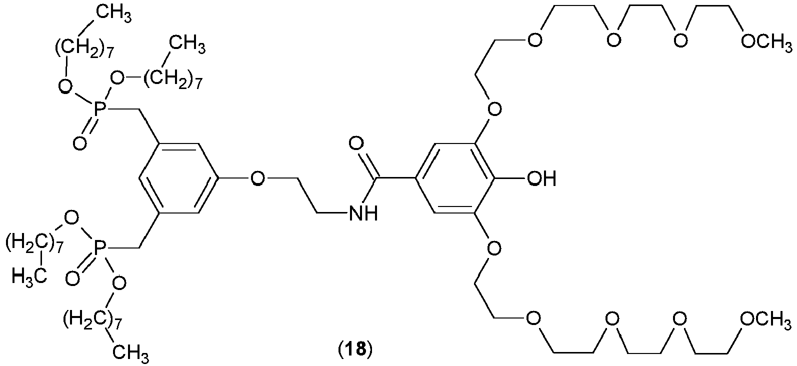

To a solution of 2.55g (1.83 mmol - 1.0 Eq.) of compound (17) in 40.0 mL of EtOAc were added 355.0 mg of Pd/C 10% as catalyst. The heterogeneous solution was purged with an atmosphere of hydrogen (five times), then vigorously stirred at RT overnight, the catalyst was filtered off over Celite, the crude product was concentrated under reduced pressure, to give 2.32compound **(18)** as a colorless oil (97%).

### Analysis:

¹H NMR (400 MHz, MeOD) δ 7.24 (s, 2H), 6.85 (m, 3H), 4.25 - 4.18 (m, 4H), 4.16 (t, J = 5.7 Hz, 2H), 4.13 - 4.05 (m, 1H), 4.06 - 3.90 (m, 8H), 3.91 - 3.84 (m, 4H), 3.78 - 3.69 (m, 6H), 3.68 - 3.58 (m, 19H), 3.55 - 3.47 (m, 4H), 3.33 (s, 6H), 3.24 - 3.13 (ABx, J = 21.9 Hz, 4H), 1.59 (m, 8H), 1.37 - 1.20 (m, 44H), 0.90 (t, J = 6.85 Hz, 12H).

¹³C NMR (101 MHz, MeOD) δ 169.79, 148.23, 141.70, 125.33, 115.98, 108.36, 72.96, 71.66, 71.58, 71.53, 71.36, 70.77, 70.03, 67.74, 67.67, 61.53, 59.09, 58.32, 40.71, 34.28, 33.00, 31.66, 31.60, 30.67, 30.40, 30.27, 26.69, 23.74, 20.86, 18.37, 14.49, 14.46.

³¹P NMR (162 MHz, MeOD) δ 27.17.

### 4.6. Step 6 - Preparation of Linker 3 (L3)

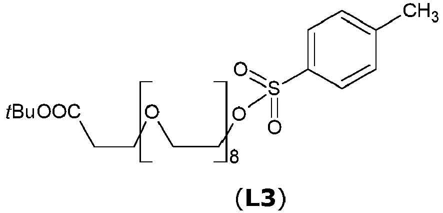

To 3.00 g (6.02 mmol - 1 Eq.) of t-Butyl 1-hydroxy-3,6,9,12,15,18,21,24-octaoxaheptacosan-27-oate in 50 mL of DCM, was added 1.69 mL (12.04 mmol - 2 Eq.) of Et₃N slowly, the mixture was stirred 10 min, then 2.32 g (12.04 mmol - 2 Eq.) de tosyl chloride was added slowly.

After the night TLC (EtP, AcOEt 7:3, KMnO₄) showed no traces of starting material. The mixture was concentrated, tosyl chloride salts were precipitated in AcOEt and filtered over celite pad, washed 3 times with AcOEt, to give the crude product (orange oil). It was then purified by Flash chromatography, Interchim-80g-50, liquid deposit in EtP/AcOEt (9:1), Eluent: 15 min EtP/AcOEt (9:1), 10 min EtP/AcOEt (8:2) with isocratic, and 10min EtP/AcOEt (7:3).

3.52 g of Linker 3 as a colourless oil was recovered (90%)

### Analysis:

¹H NMR (400 MHz, MeOD) δ 7.80 (d, J = 8.3 Hz, 2H), 7.45 (d, J = 8.2 Hz, 2H), 4.14 (t, J = 4.5 Hz, 2H), 3.77 - 3.50 (m, 32H), 2.51 - 2.43 (m, 5H), 1.45 (s, 9H).

### 4.7. Step 7 - Preparation of compound (19)

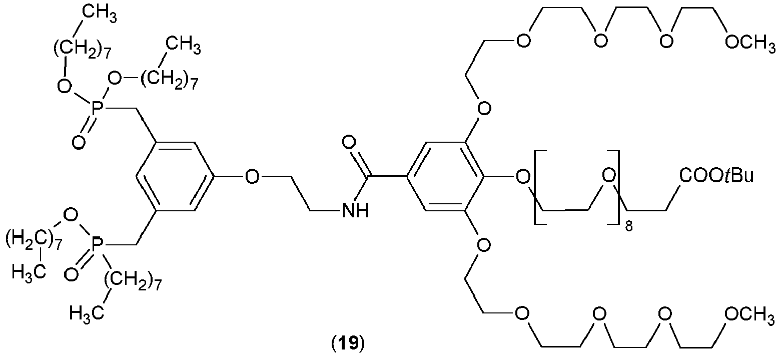

To a solution of 1.0 g (0.76 mmol - 1.0 Eq) of compound **(18)** in 25 mL of acetone, 0.48 g (0.84 mmol - 1.1 Eq.) of Linker 3, 51.3 mg (0.30 mmol - 0.4 Eq.) of KI and 340 mg (2.45 mmol - 3.2 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure. Purification by flash chromatography (CH₂Cl₂/MeOH 95/5 to 8/2 to 6/4) afforded 827 mg of pure compound **(19)** as a yellowish oil (65% yield).

### Analysis:

¹H NMR (400 MHz, MeOD) δ 7.26 (s, 2H), 6.86 (m, 3H), 4.29 - 4.22 (m, 6H), 4.18 (t, J = 5.6 Hz, 2H), 3.97 (m, 8H), 3.91 - 3.86 (m, 4H), 3.78 (m, 3H), 3.73 - 3.50 (m, 48H), 3.34 (s, 6H), 3.25 - 3.11 (ABx, J = 21.9 Hz, 4H), 2.48 (t, J = 6.2 Hz, 2H), 1.67 - 1.55 (m, 8H), 1.41 - 1.23 (m, 44H), 0.94 - 0.86 (t, J = 6.7 Hz 12H).

¹³C NMR (101 MHz, MeOD) δ 172.76, 169.27, 153.62, 140.98, 134.50, 131.15, 116.01, 107.25, 81.74, 73.60, 72.94, 71.51, 71.44, 71.29, 71.25, 71.23, 71.15, 71.10, 71.05, 70.99, 70.51, 69.57, 67.85, 67.75, 67.68, 67.51, 59.16, 58.32, 40.80, 37.18, 34.27, 32.99, 31.67, 31.61, 30.40, 30.27, 28.41, 26.69, 23.74, 18.37, 14.49.

³¹P NMR (162 MHz, MeOD) δ 27.17.

### 4.8. Step 8 - Preparation of a dendritic molecule of formula (I-D)

In a flask containing 826 mg (0.48 mmol - 1.0 Eq.) dissolved in 8 mL of DCM, 1.23 mL (15.9 mmol, 33 Eq). of trifluoroacetic acid (TFA) was added dropwise. The mixture was stirred for 2h, then evaporated under reduced pressure, affording the dendritic molecule of formula **(I-D)** as a colorless oil (91%).

### Analysis:

¹H NMR (400 MHz, MeOD) δ 7.25 (s, 2H), 6.86 (m, 3H), 4.28 - 4.20 (m, 6H), 4.17 (t, J = 5.6 Hz, 2H), 3.97 (m, 8H), 3.92 - 3.85 (m, 4H), 3.82 - 3.73 (m, 4H), 3.76 - 3.53 (m, 45H), 3.55 - 3.48 (m, 4H), 3.33 (s, 6H), 3.20 (ABx, J = 21.9 Hz, 4H), 2.54 (t, J = 6.2 Hz, 2H), 1.67 - 1.53 (m, 8H), 1.31 (m, 44H), 0.90 (t, J = 7.0 Hz, 11H).

¹³C NMR (101 MHz, MeOD) δ 167.99, 152.31, 129.42, 114.57, 106.20, 72.22, 71.56, 70.20, 70.10, 70.00, 69.92, 69.88, 69.85, 69.26, 68.43, 66.38, 66.35, 66.28, 66.13, 57.73, 39.41, 34.37, 32.87, 31.59, 30.26, 30.20, 28.99, 28.87, 25.29, 22.33, 13.08.

³¹P NMR (162 MHz, MeOD) δ 27.17.

### EXAMPLE 5: Synthesis of a dendritic molecule of formula (I-E) according to the invention

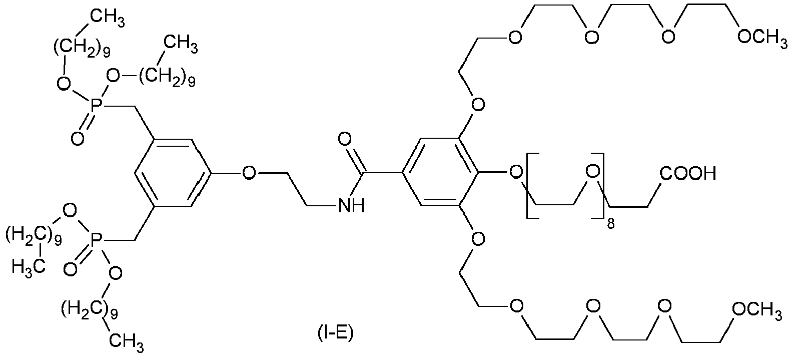

### 5.1. Step 1 - Preparation of compound (20)

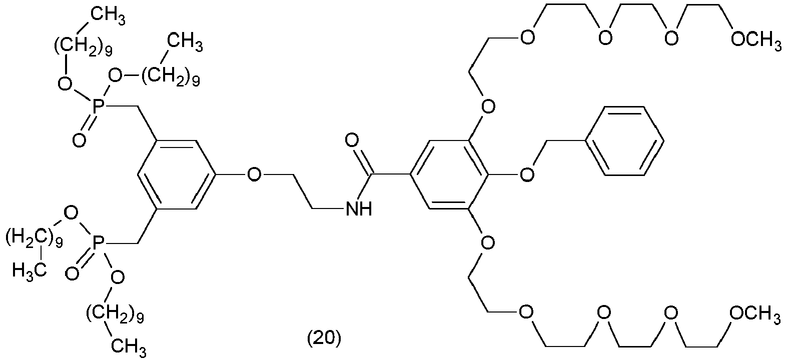

To a solution of 1.64 g (1.80 mmol - 1.0 Eq.) of compound **(10)** as prepared at step 3 of example 4 in 40 mL of EtOAc were added 960 mg (0.1 Eq.) of Pd/C 10% as catalyst. The resulting mixture was purged with an atmosphere of hydrogen (five times), then vigorously stirred at RT for 5 h.

When TLC confirmed the full consumption of compound **(10),** the catalyst was filtered over Celite and the crude product was concentrated under reduced pressure at RT. In parallel, compound **(16)** (1.1 eq, 1.98 mmol, 1.27 g) was dissolved in DCM (60.0 mL), then EDC (1.50 eq, 2.7 mmol, 529 mg), and DMAP (0.10 eq, 0.18 mmol, 22 mg) were added, and the reaction stirred for 10 min before adding the crude primary amine. The resulting mixture was stirred at RT overnight. Monitoring by TLC DCM/MeOH, 6:4, KMnO4. The reaction mixture was concentrated under reduced pressure, the crude was purified by flash chromatography

(SiO2 40.0 g, solid deposit in DCM, eluent: DCM/MeOH 100/0 to 90/10) and give expected product **(20)** (2.41 g, 88% yield) as a colorless oil

### Analysis:

¹H NMR (400 MHz, MeOD) δ 7.46 - 7.39 (s, 2H), 7.28 - 7.14 (m, 3H), 7.11 (s, 2H), 6.79 - 6.72 (m, 3H), 5.00 (s, 2H), 4.13 - 4.03 (m, 6H), 3.91 - 3.81 (m, 8H), 3.79 - 3.75 (m, 4H), 3.65 (t, J = 5.6 Hz, 2H), 3.62 - 3.59 (m, 4H), 3.55 - 3.42 (m, 17H), 3.40 - 3.36 (m, 4H), 3.21 (s, 6H), 3.09 (ABx, J = 21.4 Hz, 4H), 1.56 - 1.44 (m, 8H), 1.31 - 1.11 (m, 56H), 0.79 (t, J = 6.7 Hz, 12H).

¹³C NMR (101 MHz, MeOD) δ 169.55, 160.40, 154.06, 141.75, 139.23, 130.67, 129.73, 129.22, 128.99, 116.01, 107.50, 75.94, 72.95, 71.85, 71.67, 71.58, 71.53, 71.35, 70.84, 70.00, 67.75, 67.68, 67.55, 59.09, 54.80, 40.78, 34.31, 33.11, 32.94, 31.67, 31.61, 30.75, 30.73, 30.67, 30.52, 30.32, 26.70, 23.78, 14.50.

³¹P NMR (162 MHz, MeOD) δ 27.15.

### 5.2. Step 2 - Preparation of compound (21)

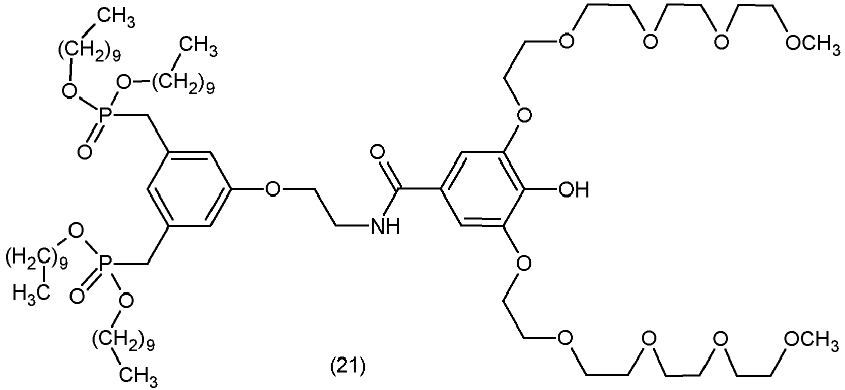

To a solution of 2.41 g (1.6 mmol - 1.0 Eq.) of compound **(20)** in 40 mL of EtOAc were added 340.0 mg of Pd/C 10% as catalyst. The heterogeneous solution was purged with an atmosphere of hydrogen (five times), then vigorously stirred at RT for 5h, the catalyst was filtered off over Celite, the crude product was concentrated under reduced pressure, to give 2.25 g of compound **(21)** as a colorless oil (99%).

### Analysis:

¹H NMR (400 MHz, MeOD) δ 7.24 (s, 2H), 6.85 (m, 3H), 4.22 - 4.20 (m, 4H), 4.16 (t, J = 5.7 Hz, 2H), 4.13 - 4.07 (m, 2H), 4.01 - 3.92 (m, 8H), 3.89 - 3.85 (m, 3H), 3.77 - 3.71 (m, 6H), 3.69 - 3.57 (m, 26H), 3.54 - 3.47 (m, 4H), 3.33 (s, 6H), 3.19 (ABx, J = 22.0 Hz, 4H), 1.66 - 1.55 (m, 8H), 1.29 (m, 56H), 0.90 (d, J = 7.3 Hz, 12H).

¹³C NMR (101 MHz, MeOD): δ 172.97, 169.77, 148.23, 141.71, 125.32, 115.99, 108.37, 72.97, 71.67, 71.58, 71.54, 71.36, 70.77, 70.03, 67.75, 67.67, 61.53, 59.10, 58.32, 40.71, 34.30, 33.12, 32.94, 31.67, 31.64, 31.61, 30.75, 30.73, 30.67, 30.52, 30.32, 26.70, 23.78, 20.86, 18.37, 14.50, 14.47.

³¹P NMR (162 MHz, MeOD) δ 27.16.

### 5.3. Step 3 - Preparation of compound (22)

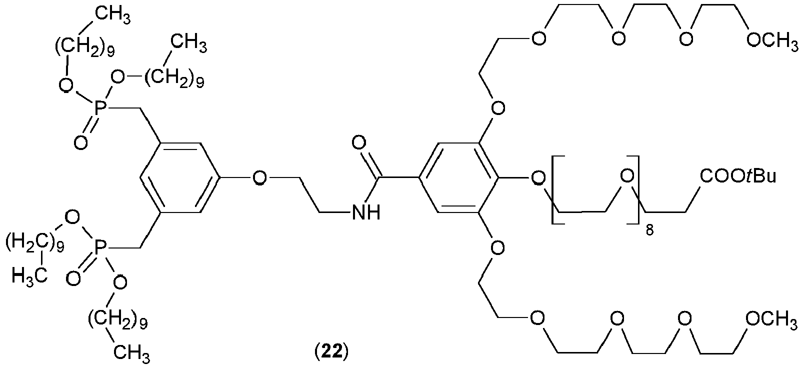

To a solution of 1.01 g (0.705 mmol - 1.0 Eq.) of compound **(21)** in 25 mL of acetone, 0.440 g (0.775 mmol - 1.1 Eq.) of Linker 3 as prepared at step 6 of example 48 mg (0.282 mmol - 0.4 Eq.) of KI and 313 mg (2.26 mmol - 3.2 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure. Purification by flash chromatography (CH₂Cl₂/MeOH 95/5 to 8/2 to 6/4) afforded 736 mg of compound **(22)** as a yellowish oil (59% yield).

### Analysis:

¹H NMR (400 MHz, MeOD) δ 7.27 (s, 2H), 6.86 (m, 3H), 4.30 - 4.22 (m, 6H), 4.18 (t, *J* = 5.6 Hz, 2H), 4.03 - 3.91 (m, 8H), 3.93 - 3.86 (m, 4H), 3.81 - 3.75 (m, 3H), 3.76 - 3.46 (m, 48H), 3.34 (s, 6H), 3.20 (ABx, J = 21.9 Hz, 4H), 2.48 (t, J = 6.2 Hz, 2H), 1.60 (m, 8H), 1.45 (s, 9H), 1.29 (s, 56H), 0.90 (t, *J* = 6.7 Hz, 12H).

¹³C NMR (101 MHz, MeOD) δ 153.63, 116.02, 107.32, 81.74, 72.95, 71.52, 71.46, 71.30, 70.53, 69.60, 67.86, 67.75, 59.15, 58.32, 37.19, 33.11, 31.67, 31.61, 30.75, 30.73, 30.51, 30.32, 28.40, 26.70, 23.77, 14.49.

³¹P NMR (162 MHz, MeOD) δ 27.16.

### 5.4. Step 4 - Preparation of a dendritic molecule of formula (I-E)

In a flask containing 736 mg (0.406 mmol - 1.0 Eq.) of compound **(22)** dissolved in DCM, 30 Eq. of TFA was added dropwise. The mixture was stirred for 2h, then evaporated under reduced pressure, affording 645 mg of the dendritic molecule of formula **(I-E)** as a colorless oil (93%)

### Analysis:

¹H NMR (400 MHz, MeOD) δ 7.15 (s, 2H), 6.76 (m, 3H), 4.18 - 4.11 (m, 6H), 4.07 (t, J = 5.6 Hz, 2H), 3.94 - 3.82 (m, 8H), 3.81 - 3.77 (m, 4H), 3.71 - 3.45 (m, 48H), 3.44 - 3.40 (m, 4H), 3.23 (s, 6H), 3.10 (ABx, J = 21.8 Hz, 4H), 2.48 (t, J = 6.2 Hz, 2H), 1.50 (q, J = 6.7 Hz, 8H), 1.30 - 1.14 (m, 56H), 0.84 - 0.73 (m, 12H).

### EXAMPLE 6: Synthesis of a dendritic molecule of formula (I-F) according to the invention

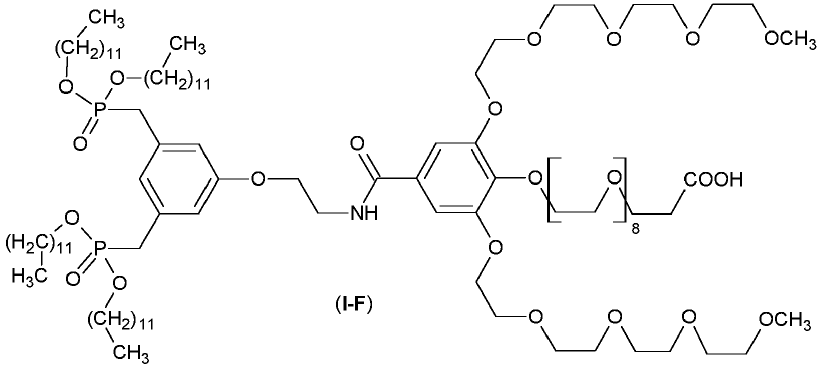

### 6.1. Step 1 - Preparation of compound (23)

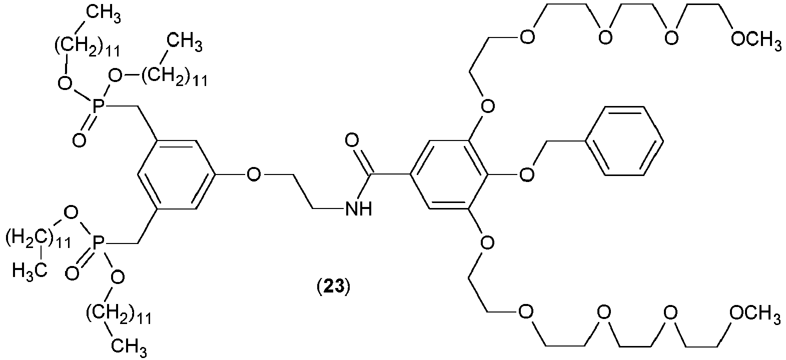

To a solution of 300 mg (0.312 mmol - 1.1 Eq.) of compound **(13),** as prepared at step 3 of example 3, in 15 mL of EtOAc were added 120 mg (0.1 Eq.) of Pd/C 10% as catalyst. The resulting mixture was purged with an atmosphere of hydrogen (five times), then vigorously stirred at RT for 5h. In a separate flask, 200 mg (0.260 mmol - 1.0 Eq.) of compound **(16),** as prepared in step 3 of example 4, were dissolved in 10 mL of CH₂Cl₂ before 0.1 mL (1.3 mmol - 3.0 Eq.) of (COCl)₂ and 4 drops of DMF were added. The orange solution was stirred at RT for 5h. When TLC confirmed the full consumption of compound **(13),** the catalyst was filtered over Celite and the crude product was concentrated under reduced pressure at RT. The acyl chloride was simultaneously concentrated under reduced pressure, dissolved in CH₂Cl₂ then the crude primary amine and 0.16 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure. Purification by flash chromatography (100% EtOAc then CH₂Cl₂/EtOAc/MeOH 80/12/8 to 6/0/4) were conducted to separate the pure and impure fractions, to afford compound **(23)** (487.2 mg, 81%) as colorless oil.

### Analysis:

¹H NMR (400 MHz, MeOD) δ 7.24 (s, 2H), 6.85 (m, 3H), 4.22 - 4.20 (m, 4H), 4.16 (t, *J* = 5.7 Hz, 2H), 4.13 - 4.07 (m, 2H), 4.01 - 3.92 (m, 8H), 3.89 - 3.85 (m, 3H), 3.77 - 3.71 (m, 6H), 3.69 - 3.57 (m, 26H), 3.54 - 3.47 (m, 4H), 3.33 (s, 6H), 3.19 (ABx, J = 22.0 Hz, 4H), 1.66 - 1.55 (m, 8H), 1.29 (m, 78H), 0.90 (d, J = 7.3 Hz, 12H).

¹³C NMR (101 MHz, MeOD) δ 173.10, 169.67, 148.33, 141.71, 125.32, 116.02, 108.47, 72.97, 71.67, 71.58, 71.54, 71.36, 70.83, 70.03, 67.75, 67.67, 61.53, 59.10, 58.32, 40.71, 34.30, 33.12, 32.94, 31.67, 31.64, 31.61, 30.75, 30.73, 30.67, 30.52, 30.32, 26.70, 23.78, 20.86, 18.37, 14.50, 14.47.

³¹P NMR (162 MHz, MeOD) δ 27.16.

### 6.2. Step 2 - Preparation of compound (24)

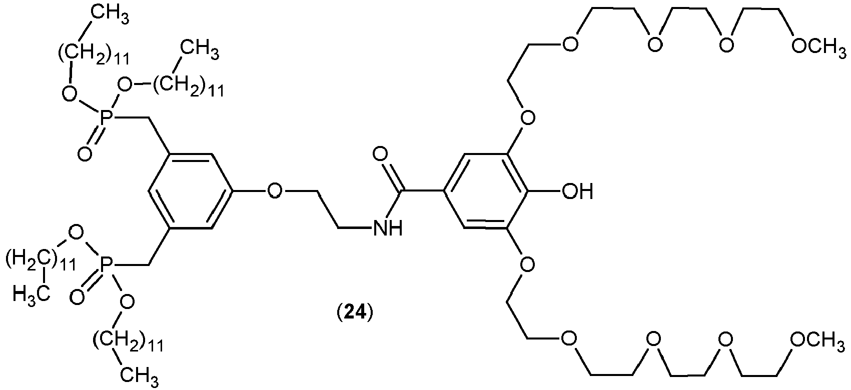

To a solution of 382 mg (0.236 mmol - 1.0 Eq.) of compound **(23)** in 40 mL of EtOAc were added 150.0 mg of Pd/C 10% as catalyst. The heterogeneous solution was purged with an atmosphere of hydrogen (five times), then vigorously stirred at RT for 5h, the catalyst was filtered off over Celite, the crude product was concentrated under reduced pressure, to give 320 mg of compound **(24)** as a colorless oil (89%).

### Analysis:

¹H NMR (400 MHz, MeOD) δ 7.24 (s, 2H), 6.85 (m, 3H), 4.25 - 4.18 (m, 4H), 4.16 (t, *J* = 5.7 Hz, 2H), 4.13 - 4.05 (m, 1H), 4.06 - 3.90 (m, 8H), 3.91 - 3.84 (m, 4H), 3.78 - 3.69 (m, 6H), 3.68 - 3.58 (m, 19H), 3.55 - 3.47 (m, 4H), 3.33 (s, 6H), 3.24 - 3.13 (ABx, J = 21.9 Hz, 4H), 1.59 (m, 8H), 1.37 - 1.20 (m, 78H), 0.90 (t, J = 6.85 Hz, 12H).

¹³C NMR (101 MHz, MeOD) δ 169.82, 148.27, 141.74, 125.30, 116.0, 108.40, 72.93, 71.71, 71.60, 71.53, 71.33, 70.77, 70.03, 67.74, 67.67, 61.53, 59.09, 58.35, 40.73, 34.27, 33.02, 31.66, 31.60, 30.67, 30.40, 30.27, 26.69, 23.74, 20.86, 18.39, 14.51, 14.46.

³¹P NMR (162 MHz, MeOD) δ 27.17.

### 6.3. Step 3 - Preparation of compound (25)

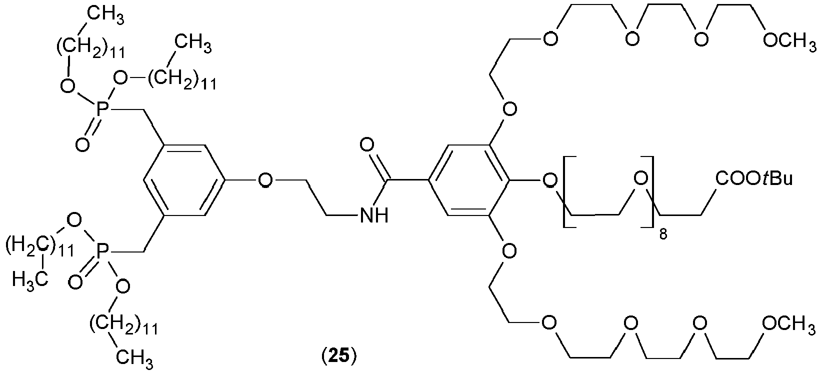

To a solution of 320 mg (0.209 mmol - 1.0 Eq.) of compound **(24)** in 40 mL of acetone, 0.145 g (0.219 mmol - 1.05 Eq.) of Linker 3 as prepared at step 6 of example 4, 33 mg (0.02 mmol - 0.1 Eq.) of KI and 45 mg (0.315 mmol - 1.5 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure to yield an orange oil. Purification by flash chromatography (CH₂Cl₂/MeOH 95/5 to 8/2 to 6/4) afforded 321 mg of compound **(25)** as a yellowish oil (76% yield).

### Analysis:

¹H NMR (400 MHz, MeOD) δ 7.27 (s, 2H), 6.86 (m, 3H), 4.30 - 4.22 (m, 6H), 4.18 (t, J = 5.6 Hz, 2H), 4.03 - 3.91 (m, 8H), 3.93 - 3.86 (m, 4H), 3.81 - 3.75 (m, 3H), 3.76 - 3.46 (m, 48H), 3.34 (s, 6H), 3.20 (ABx, J = 21.9 Hz, 4H), 2.48 (t, J = 6.2 Hz, 2H), 1.60 (m, 8H), 1.45 (s, 8H), 1.29 (s, 78H), 0.90 (t, *J* = 6.7 Hz, 12H).

¹³C NMR (101 MHz, MeOD) δ 153.63, 116.02, 107.32, 81.74, 72.95, 71.52, 71.46, 71.30, 70.53, 69.60, 67.86, 67.75, 59.15, 58.32, 37.19, 33.11, 31.67, 31.61, 30.75, 30.73, 30.51, 30.32, 28.40, 26.70, 23.77, 14.49.

³¹P NMR (162 MHz, MeOD) δ 27.16.

### 6.4. Step 4 - Preparation of a dendritic molecule of formula (I-F)

In a flask containing 321 mg (0.159 mmol - 1.0 Eq.) of compound **(25)** dissolved in DCM, 30 Eq. of TFA was added dropwise. The mixture was stirred for 2h, then evaporated under reduced pressure, affording 271mg of the dendritic molecule of formula **(I-F)** as a colorless oil (87%).

### Analysis:

¹H NMR (400 MHz, MeOD) δ 7.25 (s, 2H), 6.86 (m, 3H), 4.28 - 4.20 (m, 6H), 4.17 (t, *J* = 5.6 Hz, 2H), 3.97 (m, 8H), 3.92 - 3.85 (m, 4H), 3.82 - 3.73 (m, 4H), 3.76 - 3.53 (m, 45H), 3.55 - 3.48 (m, 4H), 3.33 (s, 6H), 3.20 (ABx, J = 21.9 Hz, 4H), 2.54 (t, J = 6.2 Hz, 2H), 1.67 - 1.53 (m, 8H), 1.31 (m, 78H), 0.90 (t, J = 7.0 Hz, 12H).

¹³C NMR (101 MHz, MeOD) δ 168.00, 152.33, 129.45, 114.59, 106.23, 72.25, 71.58, 70.22, 70.15, 70.02, 69.94, 69.86, 69.87, 69.23, 68.44, 66.39, 66.37, 66.25, 66.14, 57.69, 39.41, 34.37, 32.87, 31.59, 30.24, 30.21, 28.99, 28.85, 25.29, 22.33, 13.08.

³¹P NMR (162 MHz, MeOD) δ 27.16.

## Claims

1. A dendritic molecule of formula (I) below: 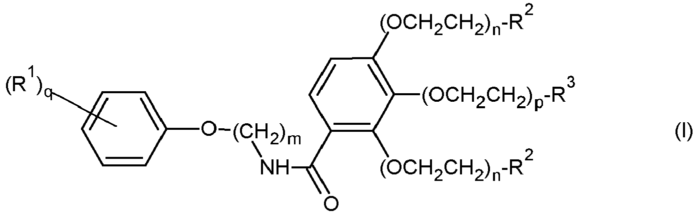 wherein :
- R¹ is a group selected among:
^{∗} an alkyl radical having at least 2 carbon atoms or an alkyl radical having at least 2 carbon atoms and comprising a terminal fluorinated group,
^{∗} a group -OR⁴ or -COOR⁴ in which R⁴ represents a linear alkyl radical having at least 4 carbon atoms or an alkyl radical having at least 2 carbon atoms and comprising a terminal fluorinated group, and
^{∗} a phosphonate group of the following formula (PG): 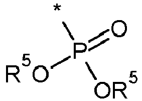 (PG) in which each of R⁵ represents a linear alkyl radical having at least 4 carbon atoms and the star represents the attachment point of said group of formula (PG) to the phenyl cycle;
- each of R² represents a linear alkyloxy radical having from 1 to 20 carbon atoms;
- R³ represents a linear alkyloxy radical having from 1 to 20 carbon atoms or a carboxyl group;
- n is an integer ranging from 1 to 16;
- p is an integer ranging from 1 to 16;
- m is an integer ranging from 1 to 4, preferably m = 1 or 2 and more preferably m = 2; and
- q is an integer ranging from 1 to 3, with the proviso that when R¹ represents a phosphonate group PG, then q = 2.

2. The dendritic molecule according to claim 1, wherein q = 2 and R¹ represents a phosphonate group of formula (PG) in which each of R⁵ represents an alkyl group having from 4 to 12 carbon atoms.

3. The dendritic molecule according to claim 2, wherein each of R⁵ represents an alkyl group selected among octyl, decanyl, and dodecanyl.

4. The dendritic molecule according to anyone of claims 1 to 3, wherein n is an integer ranging from 4 to 6 inclusively.

5. The dendritic molecule according to any one of claims 1 to 4, wherein p is an integer ranging from 4 to 10 inclusively.

6. The dendritic molecule according to any one of claims 1 to 5, wherein each of R² represents a methyloxy group.

7. The dendritic molecule according to any one of claims 1 to 6, wherein R³ represents a methyloxy group or a carboxyl group.

8. The dendritic molecule according to any one of claims 1 to 7, wherein said dendritic molecule is selected among compounds of formulae (I-A) to (I-O) whose significations of R¹ to R⁵, m, n, p and q are given in the following Table 1:
**TABLE 1**
| **Cpds** | **R¹** | **R²** | **R³** | **R⁴** | **R⁵** | **n** | **p** | **m** | **q** |
|---|---|---|---|---|---|---|---|---|---|
| (I-A) | PG | -OCH₃ | -OCH₃ | - | -(CH₂)₇-CH₃ | 4 | 4 | 2 | 2 |
| (I-B) | PG | -OCH₃ | -OCH₃ | - | -(CH₂)₉-CH₃ | 4 | 4 | 2 | 2 |
| (I-C) | PG | -OCH₃ | -OCH₃ | - | (CH₂)₁₁-CH₃ | 4 | 4 | 2 | 2 |
| (I-D) | PG | -OCH₃ | COOH | - | -(CH₂)₇-CH₃ | 4 | 9 | 2 | 2 |
| (I-E) | PG | -OCH₃ | COOH | - | -(CH₂)₉-CH₃ | 4 | 9 | 2 | 2 |
| (I-F) | PG | -OCH₃ | COOH | - | -(CH₂)₁₁-CH₃ | 4 | 9 | 2 | 2 |
| (I-G) | -(CH₂)₇-CH₃ | -OCH₃ | COOH | - | - | 4 | 9 | 2 | 2 |
| (I-H) | -(CH₂)₆CF₂CF₃ | -OCH₃ | COOH | - | - | 4 | 9 | 2 | 2 |
| (I-I) | -(CH₂)₂CF(CF₃)₂ | -OCH₃ | COOH | - | - | 4 | 9 | 2 | 2 |
| (I-J) | -COOR⁴ | -OCH₃ | COOH | -(CH₂)₇-CH₃ | - | 4 | 9 | 2 | 2 |
| (I-K) | -COOR⁴ | -OCH₃ | COOH | -(CH₂)₆CF₂CF₃ | - | 4 | 9 | 2 | 2 |
| (I-L) | -COOR⁴ | -OCH₃ | COOH | (CH₂)₂CF(CF₃)₂ | - | 4 | 9 | 2 | 2 |
| (I-M) | -OR⁴ | -OCH₃ | COOH | -(CH₂)₇-CH₃ | - | 4 | 9 | 2 | 2 |
| (I-N) | -OR⁴ | -OCH₃ | COOH | (CH₂)₂CF(CF₃)₂ | - | 4 | 9 | 2 | 2 |
| (I-O) | -OR⁴ | -OCH₃ | COOH | -(CH₂)₆CF₂CF₃ | - | 4 | 9 | 2 | 2 |

9. A process for the preparation of dendritic molecules of formula (I) as defined in claim 1 in which R¹ is a phosphonate group of formula (PG), q = 2, n = p and R² and R³ are identical and represent a linear alkyloxy radical having from 1 to 20 carbon atoms, said process comprising at least one step of reacting a compound of formula (II) below: 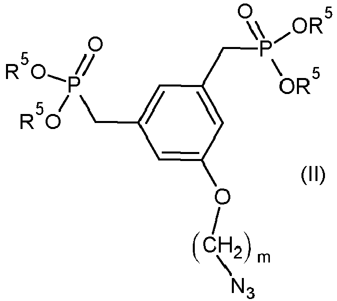 wherein R⁵ and m have the same signification as in formula (I), with a compound of formula (III) below: 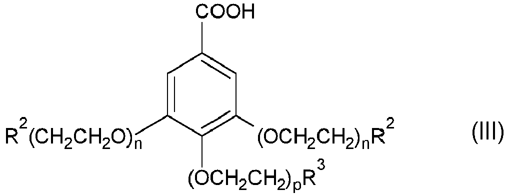 wherein n = p and have the same definition as in formula (I) and further wherein R² and R³ are identical and represent an alkyloxy group as defined in formula (I), to lead to the corresponding compound of formula (I).

10. The process according to claim 9 wherein compounds of formula (III) are prepared according to a process comprising at least the following steps:
- reacting an alcohol of formula R⁶-(OCH₂CH₂)ₙOH (IV) in which R⁶ represents a linear alkyloxy radical having from 1 to 20 carbon atoms with tosyl chloride to obtain a compound of formula (VI) below: 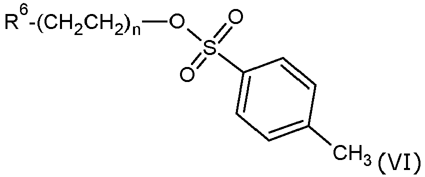 wherein R⁶ has the same meaning as in formula (IV);
- reacting said compound of formula (VI) with methyl gallate to obtain a compound of formula (VIII) below: 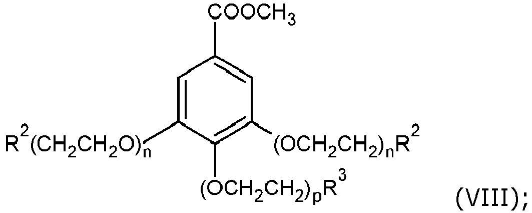 wherein n, p, R² and R³ have the same meaning as in formula (III); and
- unprotecting the carboxyl function of compound of formula (VIII) thus obtained to lead to the corresponding compound of formula (III).

11. Process according to claim 9, wherein compounds of formula (II) are prepared according to a process comprising at least the following steps:
- reacting an alcohol of formula R⁵-OH (IX) wherein R⁵ has the same meaning as in formula (I) with trimethylphosphite to obtain a compound of formula (XI) below: 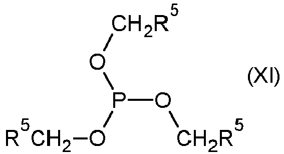 wherein R⁵ has the same meaning as in formula (I);
- reacting the compound of formula XI thus obtained with 3,5-bis(bromomethyl)phenol to obtain a compound of formula (XIII) below: 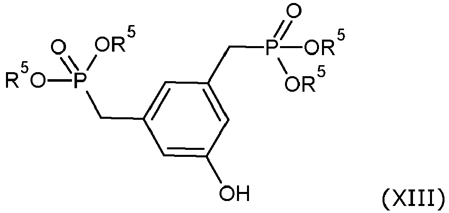 wherein R⁵ has the same meaning as in formula (I); and
- reacting the compound of formula (XIII) thus obtained with a compound of formula (XIV) below: 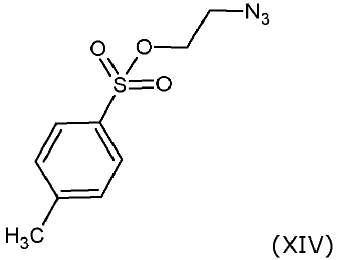 in which m has the same meaning as in formula (I) to obtain the corresponding compound of formula (II).

12. A process for the preparation of dendritic molecules of formula (I) as defined in claim 1 and in which R¹ is a phosphonate group (PG), q = 2, n and p are identical or different, R² is an alkyloxy group as defined in formula (I) and R³ is a carboxyl group, said process comprising at least the following steps:
- reacting methyl gallate with benzyl bromide to obtain a compound of formula (XV) below: 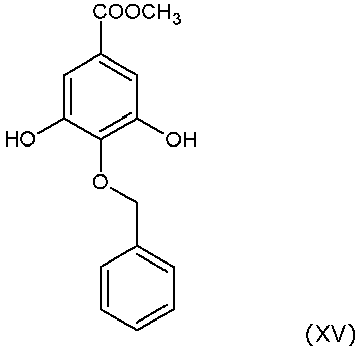
- independently reacting an alcohol of formula R²-(CH₂CH₂)ₙOH (IV') wherein R² and n have the same meaning as in formula (I) as defined in claim 1 with tosyl chloride to obtain a compound of formula (VI') below: 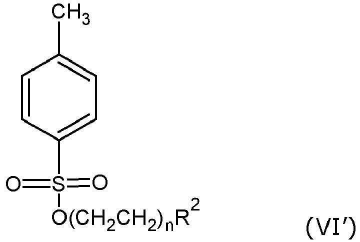 wherein R² and n have the same meaning as in formula (I) as defined in claim 1;
- reacting the compound of formula (XV) and the compound of formula (VI') thus obtained to obtain a compound of formula (XVI) below: 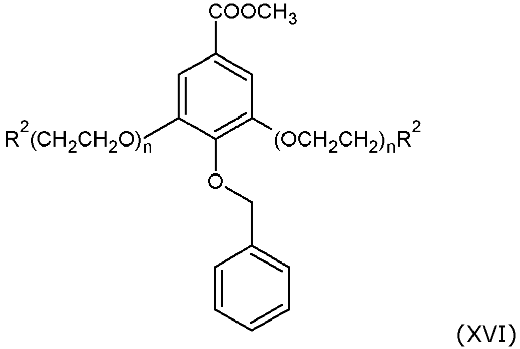 wherein R² and n have the same meaning as in formula (I) as defined in claim 1;
- unprotecting the carboxylic function of the compound of formula (XVI) thus obtained to lead to the compound of formula (XVII) below: 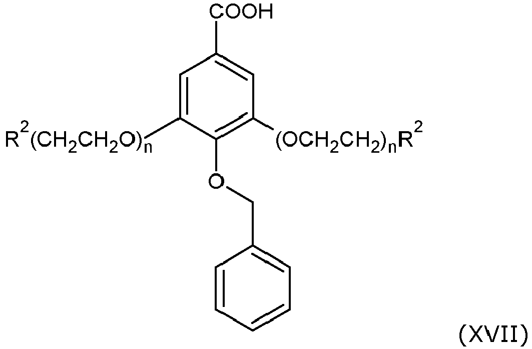 wherein R² and n have the same meaning as in formula (I) as defined in claim 1;
- reacting the compound of formula (XVII) thus obtained with a compound of formula (II) as defined in claim 10 to obtain a compound of formula (XVIII) below: 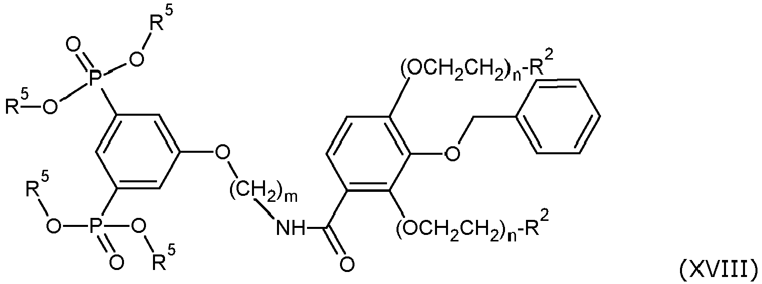 wherein R⁵, R², m and n have the same meaning as in formula (I) as defined in claim 1;
- hydrolyzing the benzyl group of the compound of formula (XVIII) thus obtained to obtain a compound of formula (XIX) below: 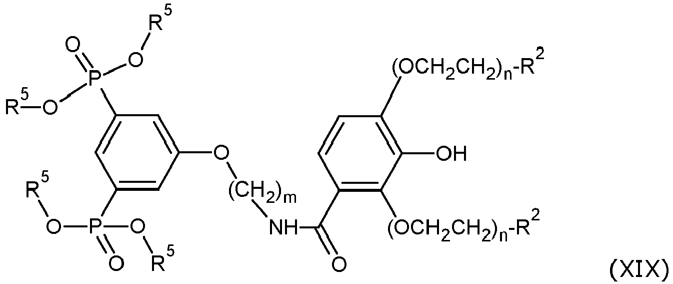 wherein R⁵, R², m and n have the same meaning as in formula (I) as defined in claim 1;
- reacting the compound of formula (XIX) thus obtained with a compound of formula (XX) below: 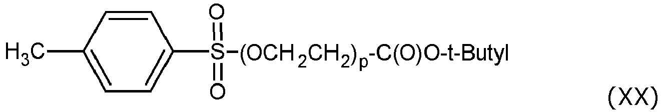 wherein p has the same meaning as in formula (I) as defined in claim 1 and has a value which is identical or different to the value of n in the compound of formula (XIX) as defined above, to obtain a compound of formula (XXI) below: 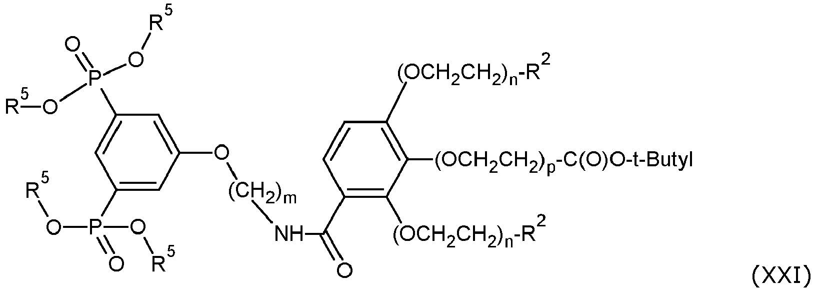 wherein R⁵, R², m, n and p have the same meaning as in formula (I) above and p has the same meaning as in formula (I) and has a value which is identical or different to the value of n in the compound of formula (XIX) as defined above;
- unprotecting the carboxylic function of compound of formula (XXI) thus obtained to lead to the corresponding dendritic molecule of formula (I).

13. The process according to claim 12, wherein compounds of formula (XX) are prepared according to a process comprising the step of reacting tosyl chloride with a compound of formula (XXII):
OHCH₂CH₂-(OCH₂CH₂)ₚ₋₁-C(O)O-*t*-Butyl (XXII)
wherein p has the same meaning as in formula (I) as defined in claim 1.

14. Use of a dendritic molecule of formula (I) as defined in any one of claims 1 to 8, as a drug carrier.

15. A dendritic molecule of formula (I) as defined in any one of claims 1 to 8, in combination with at least one imaging agent, for its use as contrast agent for medical imaging or diagnosis platforms.
